# EUROPÄISCHE PATENTSCHRIFT

(11) **EP 1 495 003 B1**
(45) Veröffentlichungstag und Bekanntmachung des Hinweises auf die Patenterteilung: **17.09.2008**
(21) Anmeldenummer: 03718715.0
(22) Anmeldetag: 28.03.2003
(51) Int. Cl.: C07D 221/20, C07D 401/04, C07D 405/04, C07D 413/04, C07D 417/04, A61K 31/4747, A61P 35/00, A61P 33/00

(54) **FREDERICAMYCIN-DERIVATE**
FREDERICAMYCIN DERIVATIVES
DERIVES DE FREDERICAMYCINE

(30) Priorität: 17.04.2002 DE 10217046
(43) Veröffentlichungstag der Anmeldung: 12.01.2005
(73) Patentinhaber: Biofrontera Discovery GmbH, 69123 Heidelberg (DE)
(72) Erfinder: WERNER, Simon, 55595 Hüffelsheim (DE); ABEL, Ulrich, 69121 Heidelberg (DE)
(74) Vertreter: Ahrens, Gabriele
(86) Internationale Anmeldenummer: PCT/EP2003/003285
(87) Internationale Veröffentlichungsnummer: WO 2003/087060

(56) Entgegenhaltungen:
- US-A- 4 584 377
- US-A- 4 673 678
- US-A- 5 166 208
- LATHAM M D ET AL: "INHIBITION OF TOPOISOMERASES BY FREDERICAMYCIN A" CANCER CHEMOTHERAPY AND PHARMACOLOGY, SPRINGER VERLAG, BERLIN, DE, Bd. 24, Nr. 3, 1989, Seiten 167-171, XP001087670 ISSN: 0344-5704

## Beschreibung

Die Erfindung betrifft neue Fredericamycin-Derivate, Arzneimittel die diese oder deren Salze enthalten, und die Verwendung der Fredericamycin-Derivate zur Behandlung von Erkrankungen, insbesondere Tumorerkrankungen.

Fredericamycin wurde 1981 aus Streptomyces griseus isoliert und zeigt Antitumoraktivität.

Fredericamycin und einige Fredericamycin-Derivate sind bekannt.

In Heterocycles 37 (1994) 1893 - 1912, J. Am. Chem. Soc. 116 (1994) 9921 - 9926, J. Am. Chem. Soc. 116 (1994) 11275 -11286, J. Am. Chem. Soc. 117 (1995) 11839 - 11849, JP 2000-072752 und in J. Am. Chem. Soc. 123 (2001) sind verschiedene, auch enantioselektive, Totalsynthesen von Fredericamycin A beschrieben.

In US 4673768 sind Alkalisalze des Fredericamycin A beschrieben. In US 4584377 Fredericamycin-Derivate, insbesondere am Ring E und F acylierte Derivate, beschrieben. In US 5,166,208 sind ebenso Fredericamycin-Derivate beschrieben, insbesondere Derivate, die am Ring F Thio- oder Amino-substituenten tragen. Die Derivate werden semisythetisch oder totalsynthetisch hergestellt.

Überraschenderweise wurde gefunden, dass Fredericamycin-Derivate, die insbesondere am Ring B oder an den Ringen A und B derivatisiert sind, potente Arzneimittel darstellen. Es wurde außerdem eine semisynthetische Möglichkeit gefunden Reste am Ring B oder an beiden Ringen A und B einzuführen, die erlauben unter anderem die Wasserlöslichkeit der Derivate zu erhöhen. Weitere aus dem Stand der Technik bekannte Wege zur Derivatisierung können an den erfindungsgemäßen Derivaten zusätzlich durchgeführt werden. Es wurde des weiteren eine Alternative gefunden Fredericamycin-Derivate wasserlöslich zu machen, in dem Cyclodextrin Einschlussverbindungen hergestellt werden.

Die Erfindung betrifft neue Fredericamycin-Derivate der allgemeinen Formel Ia oder Ib: 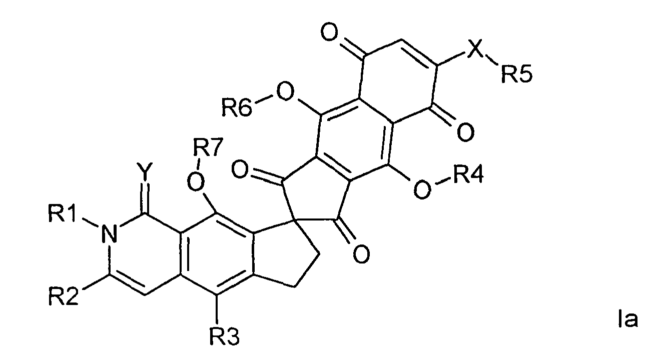 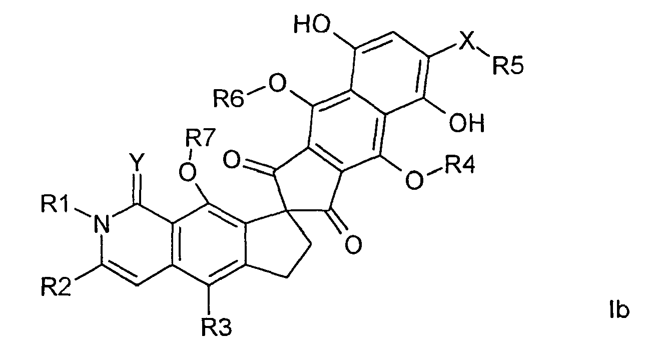 wobei jeweils
- R1: H, C₁-C₆-Alkyl, Cycloalkyl, C₁-C₄-Alkyl-Cycloalkyl,
- R2: C₁-C₁₄-Alkyl, C₂-C₁₄-Alkenyl, 1,3-Butadienyl, 1-Butan, C₁-C₄-Alkyl-Aryl, Heteroaryl, C₁-C₄-Alkyl-Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0, 1, 2, CH₂SCOR21, CH₂OSO₂-R21, CHO, CH=NOH, CH(OH)R21, -CH=NOR21, -CH=NOCOR21, -CH=NOCH₂CONR21R22, -CH=NOCH(CH₃)CONR21R22, -CH=NOC(CH₃)₂CONR21R22, -CH=N-NHCO-R23, -CH=N-NHCO-CH₂NHCOR21, -CH=N-O-CH₂NHCOR21, -CH=N-NHCS-R23, -CH=CR24R25 ( trans oder cis ), COOH, COOR21, CONR21R22, -CH=NR21, -CH=N-NR21R22, 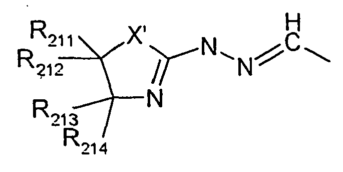 (mit X' = NR215, O, S und R211, R212, R213, R214, R215 unabhängig voneinander H oder C₁-C₆-Alkyl), -CH=N-NHSO₂-Aryl,
-CH=N-NHSO₂-Heteroaryl,
- R21, R22: unabhängig voneinander C₁-C₁₄-Alkyl, C₁-C₁₄-Alkanoyl, C₁-C₆-Alkylhydroxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbidungen (wie z.B. Glucose, Desoxyglucose, Ribose, Desoxyribose),
- R23: unabhängig von R21, die selben Bedeutungen wie R21 oder CH₂pyridinium-salze, CH2tri-C₁-C₆-alkylammonmium-salze,
- R24: unabhängig von R21, die selben Bedeutungen wie R21 oder H, CN, COCH₃, COOH, COOR21, CONR21R22, NH₂, NHCOR21
- R25: unabhängig von R21, die selben Bedeutungen wie R21 oder H, CN, COCH₃, COOH, COOR21, CONR21R22, NH₂, NHCOR21
- R24,R25: zusammen C₄-C8-Cycloalkyl,
- R3: C₂-C₁₄-Alkyl, C₂-C₁₄-Alkenyl, C₂-C₁₄-Alkinyl, Aryl, C₁-C₄-Alkyl-Aryl, Heteroaryl, C₁-C₄-Alkyl-Heteroaryl, wobei die Aryle oder Herteroaryle mit einem weiteren Aryl, C₁-C₄-Alkyl-Aryl, 0-Aryl, C₁-C₄-Alkyl-O-Aryl, Heteroaryl, C₁-C₄-Alkyl-Heteroaryl, O-Heteroaryl oder C₁-C₄-Alkyl-O-Heteroaryl substituiert sein können, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 2 bis 6, für o = 1,-1, p = 1 bis 2m+o; für m = 4 bis 6, o = -3, p = 1 bis 2m+o; Y = unabhängig von einander ausgewählt aus der Gruppe Halogen, OH, OR31, NH₂, NHR31, NR31R32, SH, SR31), CH₂NHCOR31, CH₂NHCSR31, CH₂S(O)nR31 mit n=0,1,2, CH₂SCOR31, CH₂OSO₂-R31, CHO, CH=NOH, CH(OH)R31, -CH=NOR31, -CH=NOCOR31, -CH=NOCH₂CONR31R32, -CH=NOCH(CH₃)CONR31R32, -CH=NOC(CH₃)₂CONR31R32, -CH=N-NHCO-R33, -CH=N-NHCO-CH₂NHCOR31, -CH=N-O-CH₂NHCOR31, -CH=N-NHCS-R33, -CH=CR34R35 ( trans oder cis ), COOH, COOR31, CONR31R32, -CH=NR31, -CH=N-NR31R32, 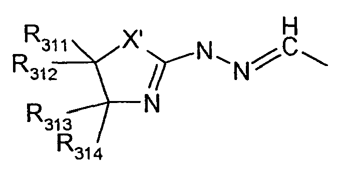 (mit X' NR315, O, S und R311, R312, R313, R314, R315 unabhängig voneinander H oder C₁-C₆-Alkyl), -CH=N-NHSO₂-Aryl,
-CH=N-NHSO₂-Heteroaryl,
- R31, R32: unabhängig voneinander C₁-C₁₄-Alkyl, C₁-C₁₄-Alkanoyl, C₁-C₆-Alkylhydroxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbidungen (wie z.B. Glucose, Desoxyglucose, Ribose, Desoxyribose),
- R33: unabhängig von R31, die selben Bedeutungen wie R31 oder CH₂pyridinium-salze, CH2tri-C₁-C₆-alkylammonmium-salze,
- R34: unabhängig von R21, die selben Bedeutungen wie R31 oder H, CN, COCH₃, COOH, COOR21, CONR31R32, NH₂, NHCOR31
- R35: unabhängig von R31, die selben Bedeutungen wie R31 oder H, CN, COCH₃, COOH, COOR31, CONR31R32, NH₂, NHCOR31
- R34,R35: zusammen C₄-C₈-Cycloalkyl,
- R5: H, C₁-C₆-Alkyl, Cycloalkyl, C₁-C₄-Alkyl-Cycloalkyl, Heterocycloalkyl, C₁-C₄-Alkyl-Heterocycloalkyl, Aryl, C₁-C₄-Alkyl-Aryl, Heteroaryl, C₁-C₄-Alkyl-Heteroaryl,
- R4,R6,R7: unabhängig voneinander H, C₁-C₆-Alkyl, CO-R41
- R41: unabhängig von R21, die selben Bedeutungen wie R21
- X: O, S, NH, N-R8, wobei R8 unabhängig von R5 die gleichen Bedeutung wie R5 annehmen kann oder R5 und R8 zusammen mit dem N eine 4, 5, 6, 7 oder 8 gliedrigen Heterocycloalkylring bilden, der optional noch ein weiteres Heteroatom ausgewählt aus der Gruppe N, O, S enthalten kann,
oder X-R5 zusammen gleich H,
- Y: O, S, NR9, wobei R9 Hoder C₁-C₆-Alkyl sein kann,
bedeutet, deren Stereoisomere, Tautomere und deren physiologisch verträglichen Salze oder Einschlussverbindungen.

Bevorzugt sind Verbindungen der Formel IIa oder IIb 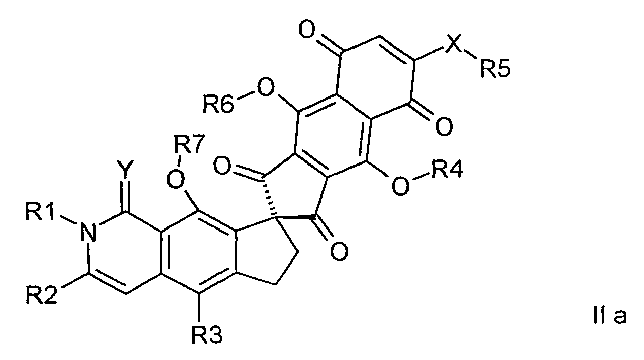 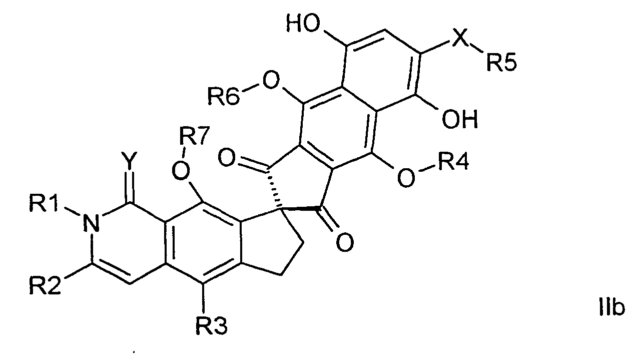 wobei die Bedeutung der Reste R1-R41, X wie oben angegeben ist, deren Tautomere und deren physiologisch verträglichen Salze oder Einschlussverbindungen.

Die Erfindung betrifft außerdem Verbindungen der Formel Ia, Ib, IIa oder IIb, bei denen die Reste R außer R3, die oben angegebenen Bedeutungen haben und R3 gegenüber R3 gleich H die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht. Die Erhöhung der Wasserlöslichkeit geschieht z.B. über die Einführung von Gruppen, die vermehrt Wasserstoffbrückenbindungen ausbilden können und/oder polar und/oder ionisch sind. Bevorzugt sind Reste R3 mit erhöhter Wasserlöslichkeit und der bei den Formeln angegebenen Bedeutung.

Die Erfindung betrifft außerdem Verbindungen der Formel Ia, Ib, IIa oder IIb, bei denen die Reste R außer R2, die oben angegebenen Bedeutungen haben und zusätzlich R2 gegenüber R2 gleich CH=CH-CH=CH-CH₃ die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht. Die Erhöhung der Wasserlöslichkeit geschieht z.B. über die Einführung von Gruppen, die vermehrt Wasserstoffbrückenbindungen ausbilden können und/oder polar und/oder ionisch sind. Ein Schlüsselzwischenprodukt sind Verbindungen mit einer Aldehyd Funktion in R2. Bevorzugt sind Reste R2 mit erhöhter Wasserlöslichkeit und der bei den Formeln angegebenen Bedeutung. Besonders bevorzugt sind Derivate mit erhöhter Wasserlöslichkeit in R2 und R3.

Bevorzugte Reste bei R2 sind Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = - 1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0,1,2, CH₂SCOR21, CH₂OSO₂-R21, CH(OH)R21, - CH=NOCOR21,
-CH=NOCH₂CONR21R22, -CH=NOCH(CH₃)CONR21R22, -CH=NOC(CH₃)₂CONR21R22, -CH=N-NHCO-R23, -CH=N-NHCO-CH₂NHCOR21, -CH=N-O-CH₂NHCOR21, -CH=N-NHCS-R23, -CH=CR24R25 ( trans oder cis ), CONR21R22, -CH=NR21, -CH=N-NR21R22, 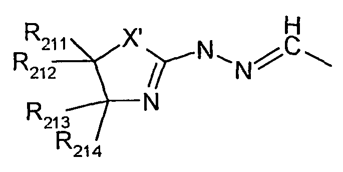 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl,

Bevorzugt sind weiterhin Verbindungen wie oben angegeben, wobei die Reste R bevorzugt unabhängig voneinander eine oder mehrere der folgenden Bedeutungen annehmen:
- R1: H, C₁-C₅-Alkyl, Cycloalkyl, insbesondere H,
- R2: C₁-C₅-Alkyl, C₁-C₄-Alkyl-Aryl, C₂-C₅-Alkenyl, Heteroaryl, C₁-C₄-Alkyl-Heteroaryl, CHF₂, CF₃, Polyolseitenkette insbesondere CHOH-CHOH-CHOH-CHOH-CH₃, CHOH-CHOH-CH=CH-CH₃, CH=CH-CHOH-CHOH-CH₃, CH₂Y (Y=F,Cl,Br,I), ), CH₂NH₂, CH₂NR21R22, CH₂NHCOR23, CH₂NHCSR23, CH₂SH, CH₂S(O)nR21 mit n=0,1,2, CH₂SCOR21, insbesondere CH₂OH, CH₂OR21, CH₂OSO₂-R21, insbesondere CHO, CH(OR21)₂, CH(SR21)₂, CN, CH=NOH, CH=NOR21, CH=NOCOR21, CH=N-NHCO-R23, CH=CR24,R25 ( trans oder cis ), insbesondere COOH (insbesondere deren physiologisch verträglichen Salze), COOR21, CONR21R22, -CH=NR21, -CH=N-NR21R22, 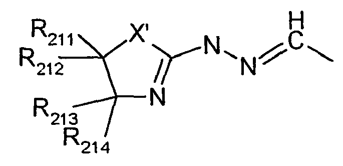 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl, CH=N-NHCO-R23,
- R21, R22: unabhängig voneinander C₁-C₆-Alkyl, Cycloalkyl, Aryl, C₁-C₄-Alkyl-Aryl, Heteroaryl, C₁-C₄-Alkyl-Heteroaryl
- R23: unabhängig von R21, die selben Bedeutungen wie R21 oder CH₂pyridinium-salze, CH2tri-C₁-C₆-alkylammonmium-salze,
- R24: unabhängig von R21, die selben Bedeutungen wie R21 oder H, CN, COCH₃, COOH, COOR21, CONR21R22, NH₂, NHCOR21
- R25: unabhängig von R21, die selben Bedeutungen wie R21 oder H, CN, COCH₃, COOH, COOR21, CONR21R22, NH₂, NHCOR21
- R24,R25: zusammen C₄-C8-Cycloalkyl,
- R3: C₂-C₁₄-Alkyl, C₂-C₁₄-Alkenyl, C₂-C₁₄Alkinyl, Aryl, C₁-C₄-Alkyl-Aryl, Heteroaryl, C₁-C₄-Alkyl-Heteroaryl, wobei die Aryle oder Herteroaryle mit einem weiteren Aryl, C₁-C₄-Alkyl-Aryl, O-Aryl, C₁-C₄-Alkyl-O-Aryl, Heteroaryl, C₁-C₄-Alkyl-Heteroaryl, O-Heteroaryl oder C₁-C₄-Alkyl-O-Heteroaryl substituiert sein können,
- R5: H, C₁-C₃-Alkyl, Cycloalkyl
- R4,R6,R7: unabhängig voneinander H, C₁-C₅-Alkyl, CO-R41
- R41: unabhängig von R21, die selben Bedeutungen wie R21
- X: O, S, NH, N-R8, insbesondere O
- Y: O, S, NH, insbesondere O
bedeutet, deren Stereoisomere, Tautomere und deren physiologisch verträglichen Salze oder Einschlussverbindungen.

Ganz besonders bevorzugt sind die Verbindungen, deren Stereoisomere, Tautomere und deren physiologisch verträglichen Salze oder Einschlussverbindungen, ausgewählt aus der Gruppe bestehend aus den Verbindungen der Beispiele 7 - 10 und den Verbindungen, die Kombinationen der verschiedenen Substituenten der Verbindungen dieser Beispiele aufweisen.

Bevorzugt sind außerdem Arzneimittel enthaltend obige Verbindungen der Formel I oder II neben den üblichen Träger und Hilfsstoffen.

Bevorzugt sind auch die oben genannten Arzneimittel in Kombination mit weitere Wirkstoffen zur Tumorbehandlung.

Diese erfindungsgemäßen Verbindungen werden zur Herstellung von Arzneimitteln zur Behandlung von Tumoren, insbesondere von solchen, die durch die Inhibierung der Topoisomerasen I und/oder II behandelt werden können, verwendet. Tumoren, die mit den erfindungsgemäßen Substanzen behandelt werden können sind z.B. Leukemie, Lungenkrebs, Melanome, Prostatatumore und Colontumore.

Des weiteren können die erfindungsgemäßen Verbindungen zur Herstellung von Arzneimitteln zur Behandlung von Neurodermitis, Parasiten und zur Immunsuppression verwendet werden.

In der Beschreibung und den Ansprüchen gelten für die einzelnen Substituenten folgende Definitionen:

Der Term "Alkyl" für sich oder als Teil eines anderen Substituenten bedeutet ein lineares oder verzweigtes Alkylketten-Radikal der jeweils angegebenen Länge. So bedeutet C₁₋₄-Alkyl z.B. Methyl, Ethyl, 1-Propyl, 2-Propyl, 2-Methyl-2-propyl, 2-Methyl-1-propyl, 1-Butyl, 2-Butyl, C₁₋₆-Alkyl z.B. C₁₋₄-Alkyl, Pentyl, 1-Pentyl, 2-Pentyl, 3-Pentyl, 1-Hexyl, 2-Hexyl, 3-Hexyl, 4-Methyl-1-pentyl oder 3,3-Dimethyl-butyl.

Der Term "C₁-C₆-Alkylhydroxy" für sich oder als Teil eines anderen Substituenten bedeutet ein lineares oder verzweigtes Alkylketten-Radikal der jeweils angegebenen Länge, und eine OH Gruppe trägt, z.B. Hydroxymethyl, Hydroxyethyl, 1-Hydroxypropyl, 2-Hydroxypropyl.

Der Term "Alkenyl" für sich oder als Teil eines anderen Substituenten bedeutet ein lineares oder verzweigtes Alkylketten-Radikal mit eine oder mehreren C=C-Doppelbindungen der jeweils angegebenen Länge, wobei mehrere Doppelbindungen bevorzugt konjugiert sind. So bedeutet C₂₋₆-Alkenyl z.B. Ethenyl, 1-Propenyl, 2-Propenyl, 2-Methyl-2-propenyl, 2-Methyl-1-propenyl, 1-Butenyl, 2-Butenyl, 1,3-Butdienyl, 2,4-Butdienyl, 1-Pentenyl, 2-Pentenyl, 3-Pentenyl, 1,3-Pentdienyl, 2,4-Pentdienyl, 1,4-Pentdienyl, 1-Hexenyl, 2-Hexenyl, 1,3-Hediexyl, 4-Methyl-1-pentenyl oder 3,3-Dimethyl-butenyl.

Der Term "Alkinyl" für sich oder als Teil eines anderen Substituenten bedeutet ein lineares oder verzweigtes Alkylketten-Radikal mit eine oder mehreren C-C-Dreifachbindungen der jeweils angegebenen Länge, wobei auch zusätzliche Doppelbindungen vorliegen können. So bedeutet C₂₋₆-Alkinyl z.B. Ethinyl, 1-Propinyl, 2-Propinyl, 2-Methyl-2-propinyl, 2-Methyl-1-propinyl, 1-Butinyl, 2-Butinyl, 1-Pentinyl, 2-Pentinyl, 3-Pentinyl, 1,4-Pentdiinyl, 1-Pentin-4-enyl, 1-Hexinyl, 2-Hexinyl, 1,3-Hexdiinyl, 4-Methyl-1-pentinyl oder 3,3-Dimethyl-butinyl.

Der Term "Halogen" steht für Fluor, Chlor, Brom, Jod, bevorzugt Brom und Chlor.

Der Term "Cycloalkyl" für sich oder als Teil eines anderen Substituenten beinhaltet gesättigte, cyclische Kohlenwasserstoffgruppen, mit 3 bis 8 C-Atomen wie z.B. Cyclopropyl, Cyclobutyl, Cyclopentyl, Cyclohexyl, 4-Methylcyclohexyl, Cyclohexylmethylen, Cycloheptyl oder Cyclooctyl.

Der Term "Heterocycloalkyl" für sich oder als Teil eines anderen Substituenten beinhaltet Cycloalkylgruppen worin bis zu zwei CH₂-Gruppen durch Sauerstoff-, Schwefel- oder Stickstoffatome ersetzt sein können und eine weitere CH₂-gruppe durch eine Carbonylfunktion ersetzt sein kann, z.B. Pyrrolidin, Piperidin, Morpholin oder

| | | | |
|---|---|---|---|
| 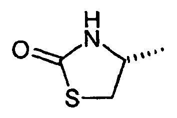 | 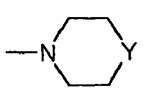 | Y=CH2, S, O, NH, NC1-C6-Alkyl | 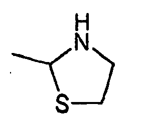 |

Der Term "Aryl" für sich oder als Teil eines anderen Substituenten beinhaltet aromatische Ringsysteme mit bis zu 3 Ringen, bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COCH, COOR11, CONH2, CONR11R12, CHO, CH=NO-C₁-C₁₀-Alkyl, C₁-C₁₀-Alk-1-enyl, NH₂, NHR11, NR11R12, Halogen haben können, wobei die Reste R11, R12 unabhängig von einander C₁-C₁₀-Alkyl, Cycloalkyl, C₁-C₄-Alkyl-Cycloalkyl, bedeuten können.

Bevorzugte Aryle sind neben Phenyl und 1-Naphtyl und 2-Naphtyl:

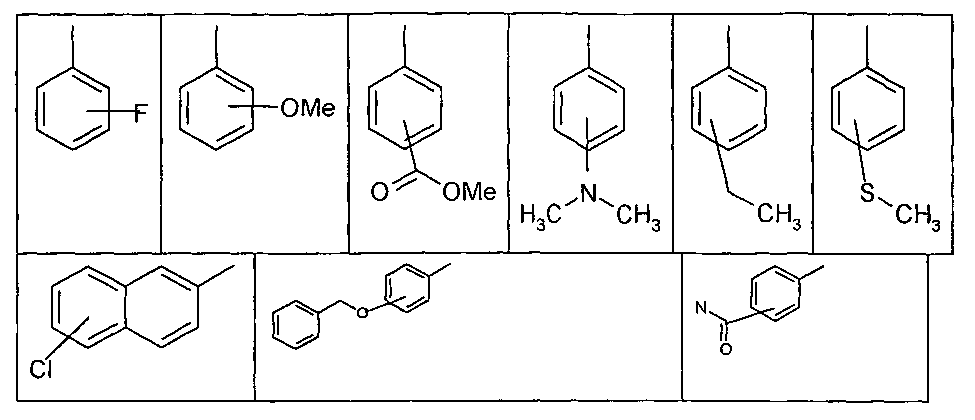

Der Term "Heteroaryl" für sich oder als Teil eines anderen Substituenten beinhaltet aromatische Ringsysteme mit bis zu 3 Ringen, und bis zu 3 gleichen oder verschiedenen Heteroatomen N, S, O bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, CONH2, CONR11R12, CHO, CH=NO-C₁-C₁₀-Alkyl, C₁-C₁₀-Alk-1-enyl, NH₂, NHR11, NR11R12, Halogen haben können, wobei die Reste R11, R12 unabhängig von einander C₁-C₁₀-Alkyl, Cycloalkyl, C₁-C₄-Alkyl-Cycloalkyl, bedeuten können.

Bevorzugte Heteroaryle sind:

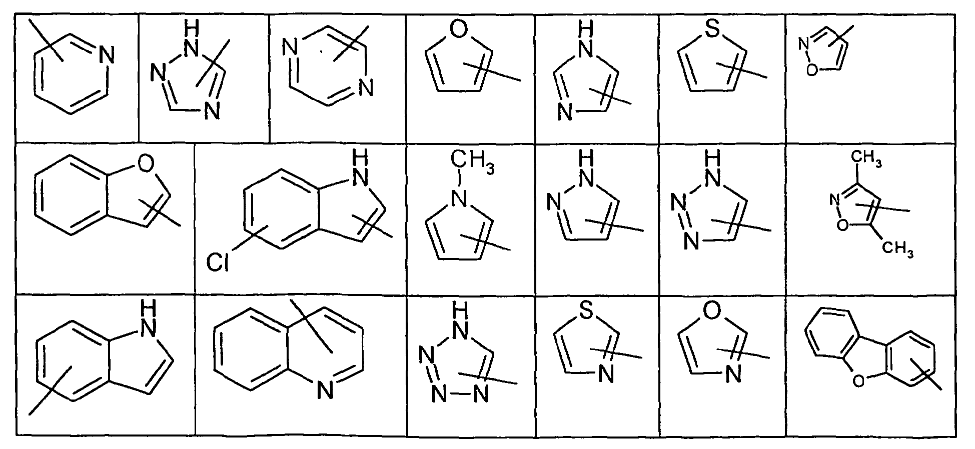

Insbesondere sind 2-furyl, 3-furyl, 2-thiophenyl, 3-thiophenyl, 3-pyridinyl, 4-pyridinyl, 4-isoxazolyl, 2-N-methylpyrrolyl, und 2-pyrazinyl bevorzugt. Ganz besonders bevorzugt sind diese als Rest R3.

Der Term "Ringsystem" bezieht sich im Allgemeinen auf 3, 4, 5, 6, 7, 8, 9 oder 10 gliedrige Ringe. Bevorzugt sind 5 und 6 gliedrige Ringe. Des weiteren sind Ringsysteme mit einem oder 2 anellierten Ringen bevorzugt.

Die Verbindungen der Formel I können als solche oder falls sie acidische oder basische Gruppen aufweisen in Form ihrer Salze mit physiologisch verträglichen Basen oder Säuren vorliegen. Beispiele für solche Säuren sind: Salzsäure, Zitronensäure, Trifluoressigsäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin. Beispiele für Basen sind Alkaliionen, bevorzugt Na, K, Erdalkaliionen, bevorzugt Ca, Mg, Ammoniumionen.

Die erfindungsgemäßen Verbindungen können in üblicher Weise oral verabfolgt werden. Die Applikation kann auch i.v., i.m., mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 0.1 µg/kg und 1 g/Kg bei oraler Gabe. Diese Dosis kann in 2 bis 4 Einzeldosen oder einmalig am Tag als Slow-release-Form gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Lösungen, oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Experimenteller Teil

Fredericamycin A ist fermentativ oder totalsynthetisch nach den bekannten Methoden zugänglich. Die reduzierten Formen der Formel I b und II b lassen sich durch milde Reduktionsmittel aus den entsprechenden Verbindungen der Formel I a und II a herstellen.

### Herstellung der Substanzen

### Substitution am B-Ring

### Palladiumkatalysierte C-C Verknüpfung

Fredericamycin (1) lässt sich mit Halogenierungsmitteln wie N-Bromsuccinimid (NBS) und N-Jodsuccinimid (NIS) in guten Ausbeuten zu den 5-Brom- bzw. 5-Jod Fredericamycin Derivaten (2) und (3) umsetzen (Schema 1).

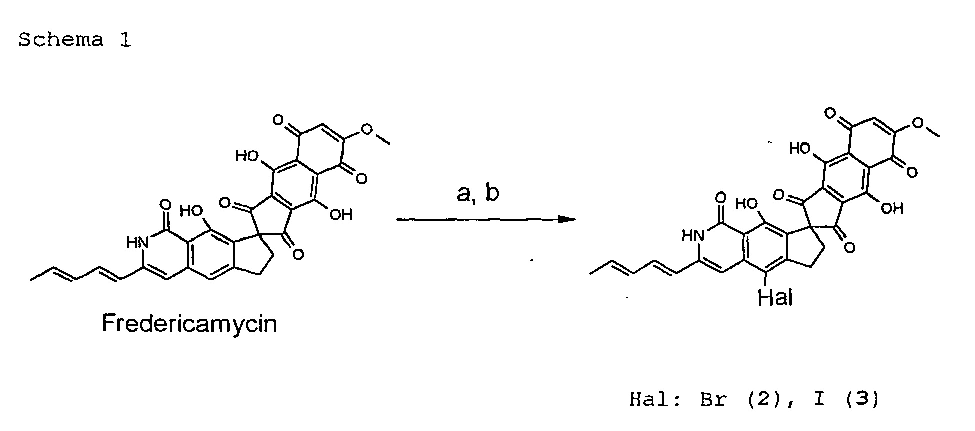

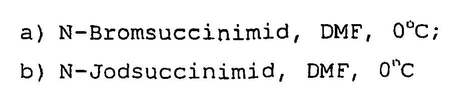

Durch palladiumkatalysierte Kreuzkupplungen, nach Suzuki, Stille bzw. nach Heck, mit Organoboronverbindungen bzw. Zinnverbindungen wie z. B.: trans-1-hexen-1yl-boronsäure (4),Phenylboronsäure (5) und 4-Fluorophenylboronsäure (6) sind die entsprechenden C-C verknüpften Fredericamycinderivate (7), (8) und (9) zugänglich. ( s. Schema 2 ).

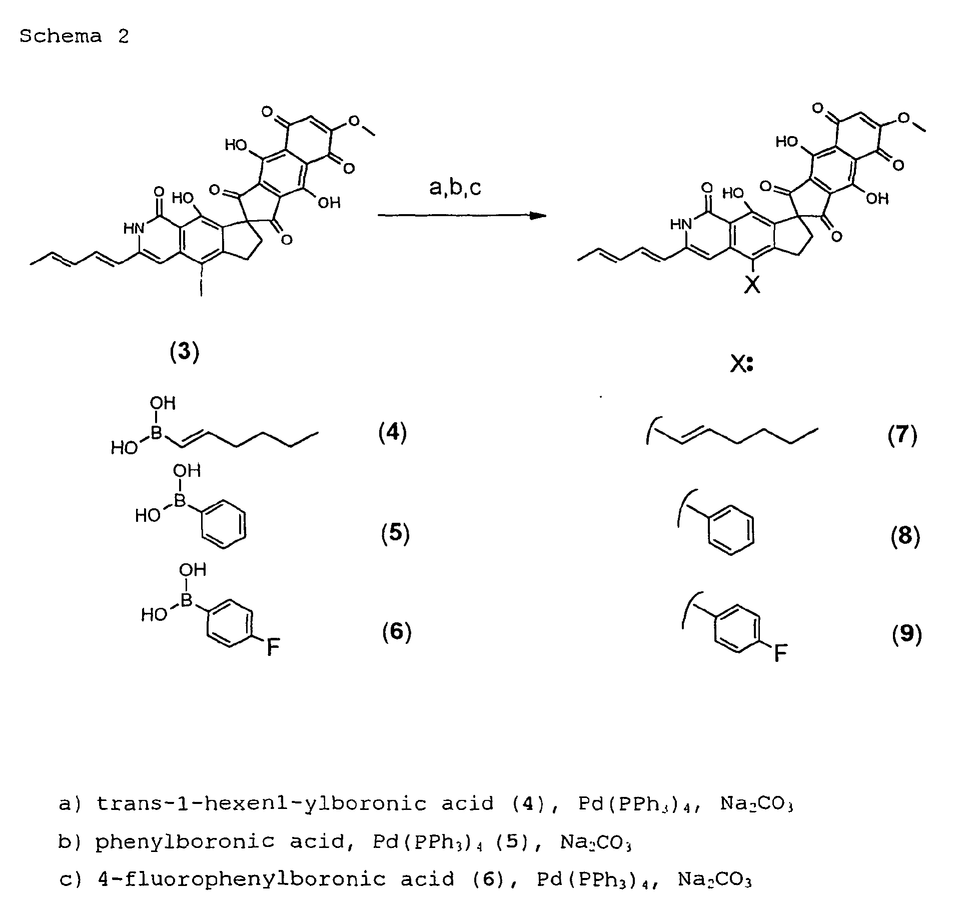

In Analogie zu Schema 3 und 4 kann auch Derivate mit X gleich einer Aldehydfunktion hergestellt werden. Beispielsweise für die Sequenz X entsprechend 1) Br, 2) Pentadienyl, 3) Tetrol, 4) Aldehyd. Über die Aldehydfunktion sind dann die weiteren erfindungsgemäßen Derivatisierungen möglich.

Für die Synthese weiterer wasserlöslichen Fredericamycin Derivaten wurde Fredericamycin (1) zunächst mit Osmium(IV)oxid an der Dienseitenkette hydroxyliert. ( s. Schema 3 ).

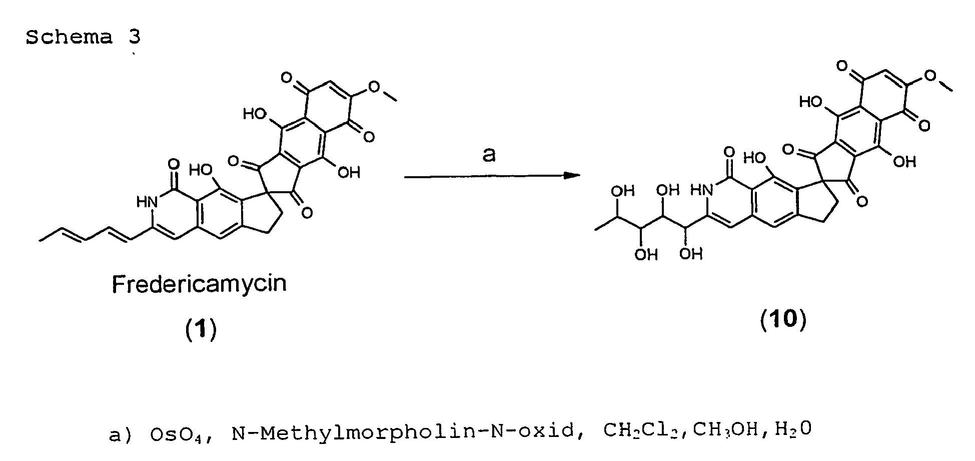

Das Fredericamycin-tetrol (10) dient ebenfalls als wichtige Zwischenstufe für die Synthese der in diesem Patent genannten Fredericamycin Derivate mit erhöhtem Löslichkeit und/oder Wirkprofil. Durch Jodatspaltung mit Natriummetaperjodat bzw. trägergebundenem Perjodat läßt sich die Tetrolseitenkette in sehr hohen Ausbeuten zum Fredericamycin-aldehyd (11) abbauen (s. Schema 4) .

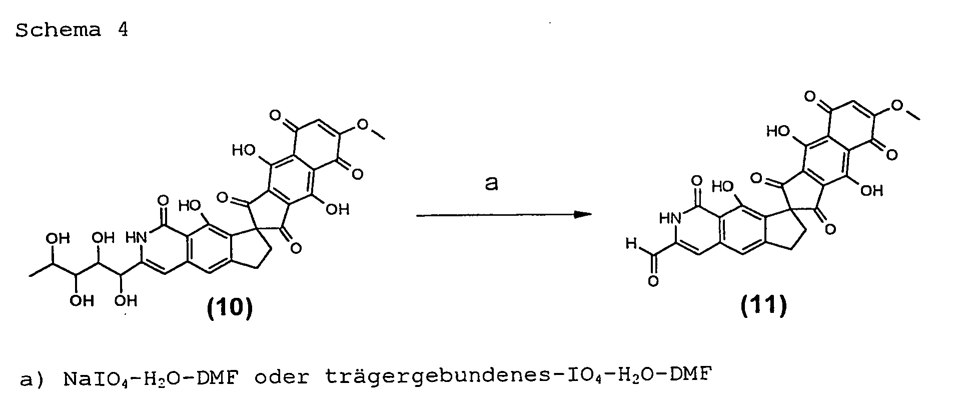

Dieser Aldehyd läßt sich durch Bromierungsreagenzien wie N-Bromsuccinimid, Brom oder andere bromgenerierende Reagenzien zur kernbromierten Verbindung (12) umsetzen ( s. Schema 5).

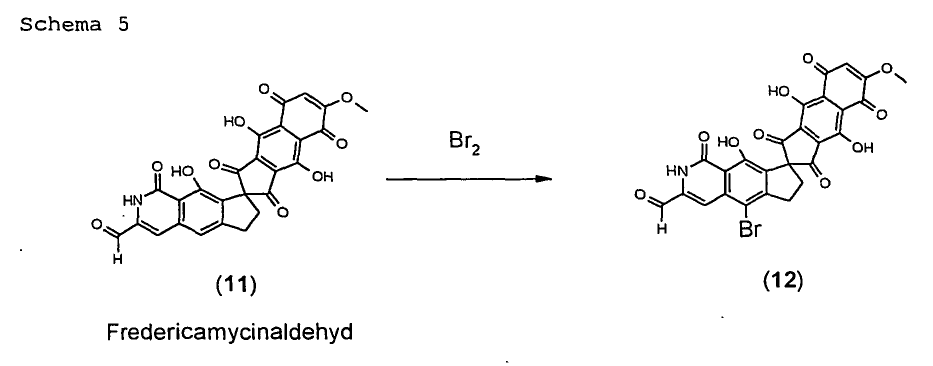

Überraschenderweise hat sich auch gezeigt, dass bei der oben beschriebenen Diolspaltung [ (10) → (11) ] in einer Stufe der kernjodierte Fredericamycin aldehyd (13) entsteht. Diese überraschende Reaktion wird nur beobachtet, wenn man Dimethylsulfoxid (DMSO) anstelle von Dimethylformamid (DMF) als Lösungsmittel verwendet ( s. Schema 6).

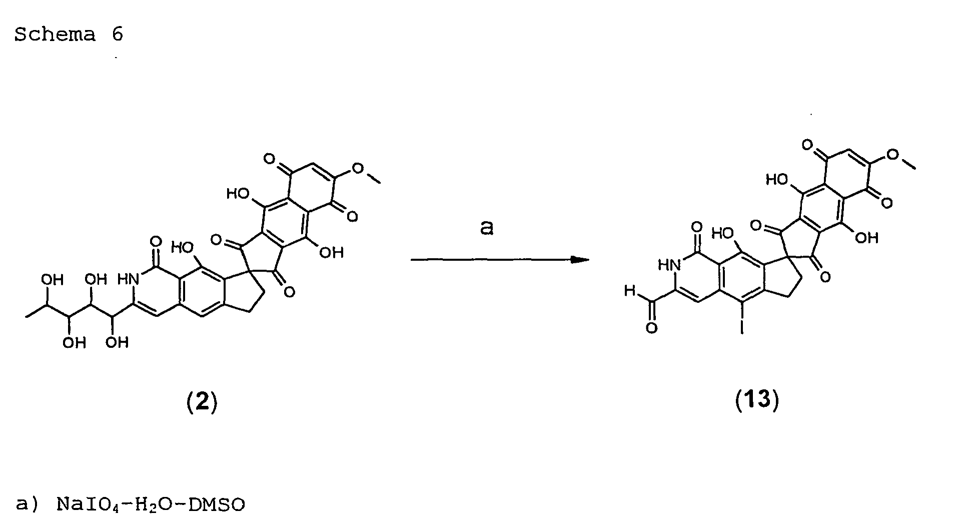

Der jodierte Fredericamycinaldehyd (13) ist wie der bromierte Fredericamycinaldehyd (12)ebenfalls für den Aufbau von Substanzbibliotheken geeignet.

Als Beispiel einer Substanzbibliothek kann der Aldehyd (12)z.B. mit Hydroxylaminen und Hydrazinen und einer nachgeschalteten Pd- katalysierten C-C Kupplung zu den entsprechenden R3 substituierten Oximen umgesetzt werden( s. Schema 7 ).

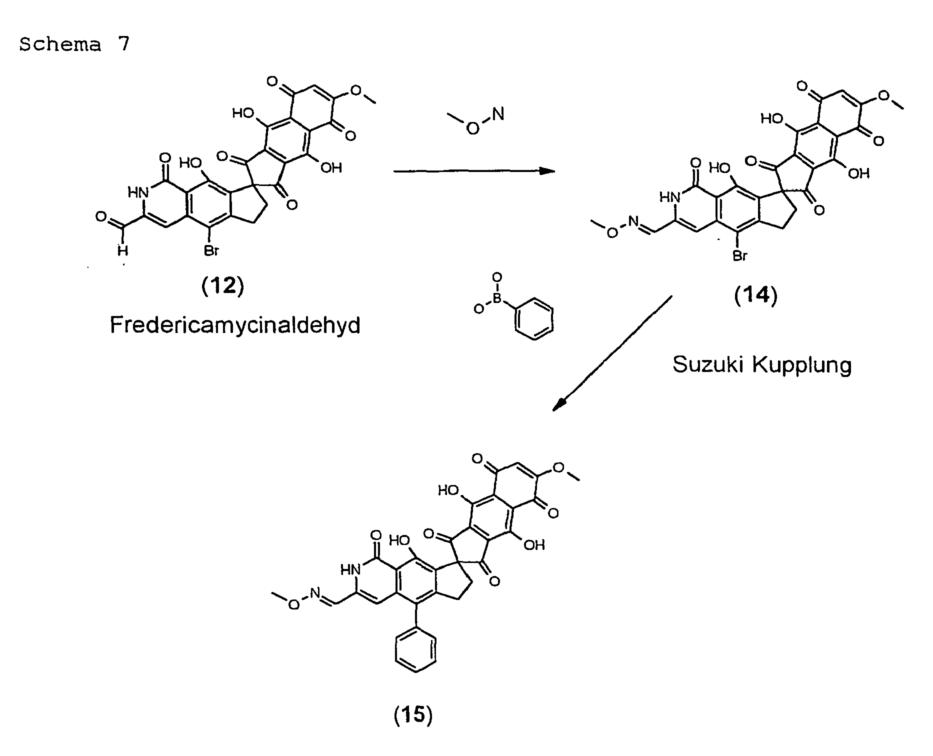

In den folgenden Schematas wird anhand von Fredericamycin und dessen Derivaten gezeigt, wie man zu erfindungsgemäßen Derivaten in Analogie kommen kann.

Die Verbindung (24) ist die Vorstufe eines N-Methylierten Fredericamycinderivates ( Schema 8 ).

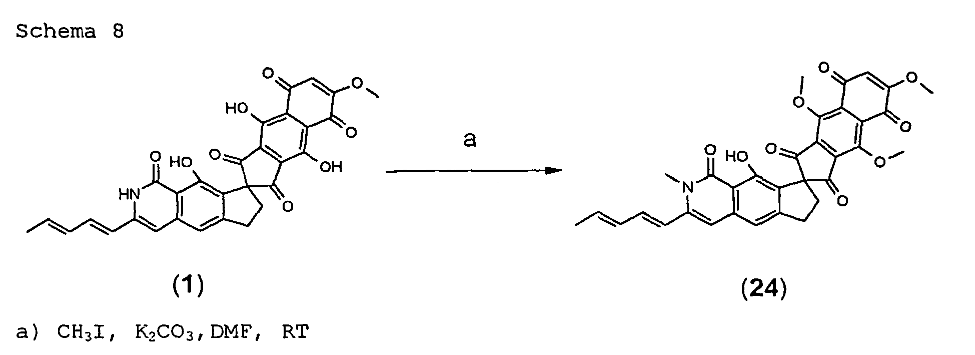

Fredericamycin kann durch Palladium/Wasserstoff nahezu quantitativ in das Tetrahydro Fredericamycin 25 umgewandelt und nach den oben beschriebenen Methoden kernhalogeniert z.B. zur Bromverbindung 26 umgesetzt werden (Schema 9):

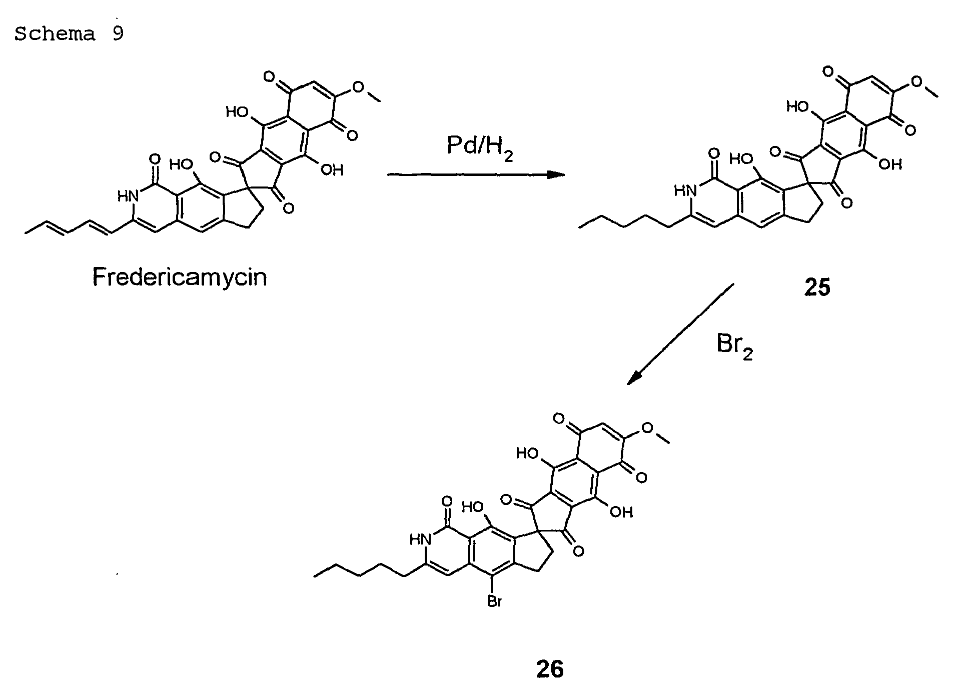

Überraschend wurde auch gefunden, dass sich die Methoxygruppierung im Fredericamycin und den erfindungsgemäßen Derivaten unter Alkali-und Erdalkaliacetat Katalyse durch Sauerstoff Nukleophile wie Alkohole oder Polyole austauschen lässt. Dabei können die können die Alkohole eine Vielzahl verschiedener Substituenten tragen.

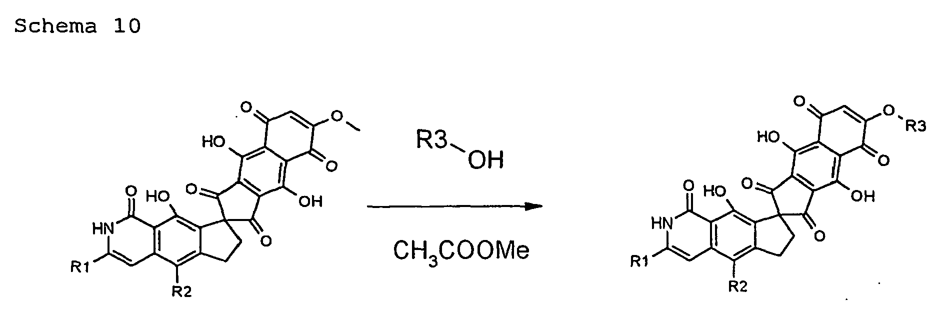

### Austausch der Methoxygruppe am F Ring

Der Austausch der Methoxygruppierung am F-Ring des Fredericamycins sowie an den Derivaten ist durch primäre, sekundäre oder aromatische Amine möglich. Dabei werden die Komponenten mit den entsprechenden primären oder sekundären Aminen bei Raumtemperatur in DMF oder einem anderen inerten Lösungsmittel gerührt. Bei aromatischen Aminen ist eine Katalyse mit Lewissäuren wie Zinn(IV)chlorid etc. erforderlich.

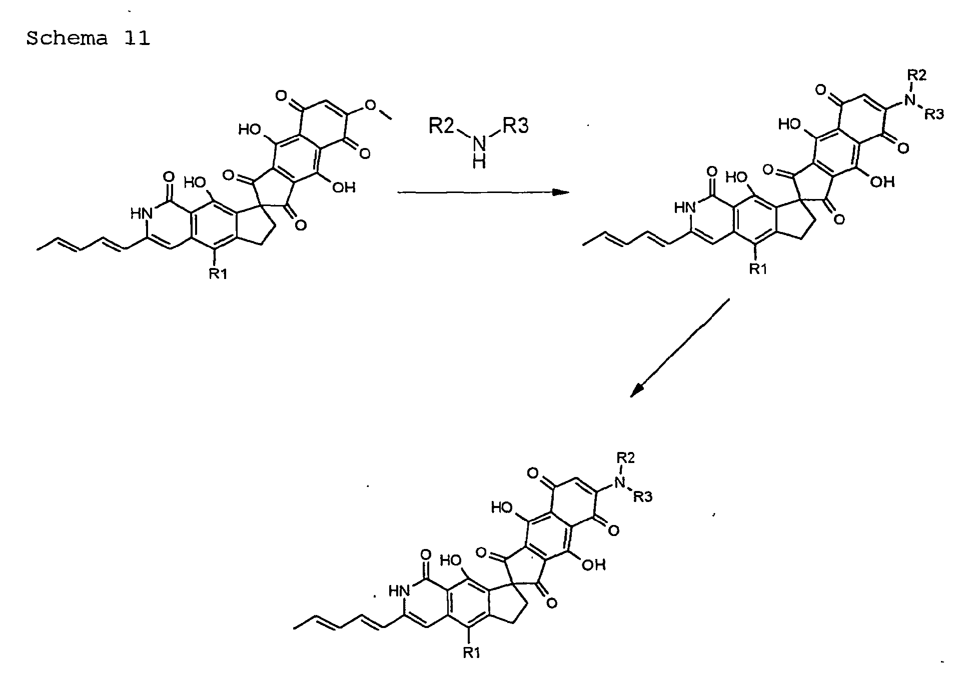

### Herstellung thioanaloger Fredericamycin Derivate

Durch Beschwefelung von Fredericamycin oder seine Derivate mit Lawesson Reagenz oder P₄S₁₀ in Pyridin sind die Thiopyridon analogen Derivate zugänglich (s. Schema 12)

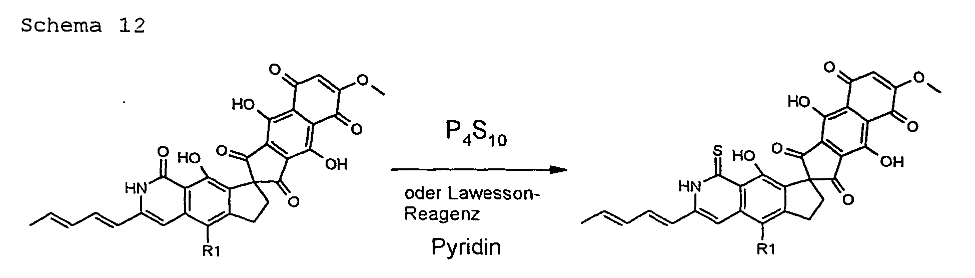

Fredericamycin (1) bildet mit Polyzuckern wie α-Cyclodextrin Einschlussverbindungen, wie (22), die gegenüber der Ausgangssubstanz gut wasserlöslich sind.

Die Dextrineinschlussverbindungen bilden sich leicht, wenn man die Komponenten im entsprechenden stöchiometrischen Verhältnis in einem geeigneten Lösungsmittel wie DMSO mischt.

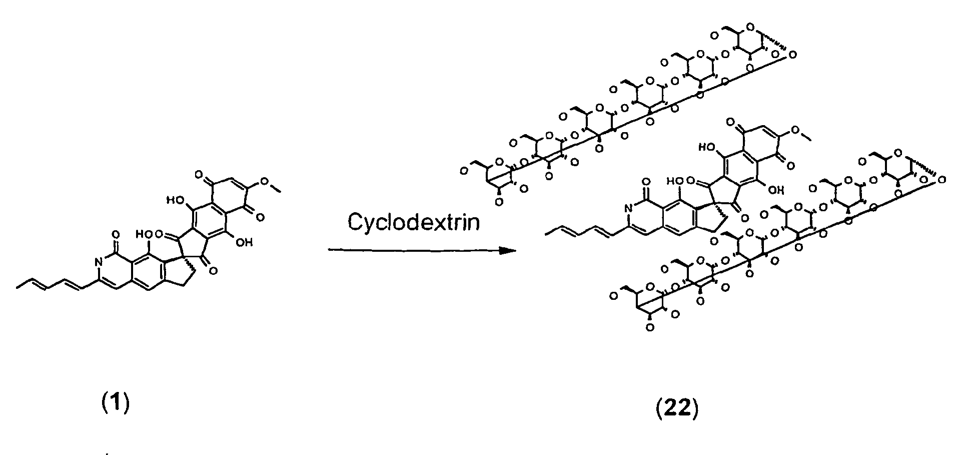

### Beispiele

### Beispiel 1

### 1-Deoxy-5-C-[(8R)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]-naphtalen]-3-yl]pentitol (10)

200mg (0.38 mmol) Fredericamycin A (1) werden in 30ml Dichlormethan gelöst. Nach der Zugabe von 20ml Methanol und 4.4ml Wasser werden 350mg ( 2.6mmol ) N-methylmorpholin-N-oxid eingetragen. Unter kräftigem Rühren tropft man 0.2ml einer 2.5%igen Osmium(IV)oxid-Lösung in t-Butanol zu. Man säuert mit die Reaktionsmischung mit 2-3 Tropfen Trifluoressigsäure an. Nach 48 stündigem Rühren ist die Reaktion laut HPLC-Kontrolle ( RP18, Acetonitril-Wasser (0.2%Essigsäure ) vollständig. Die Reaktionsmischung wird unter kräftigem Rühren in 400ml Wasser eingetragen und der dunkelrote kristalline Feststoff über einen Filter abgesaugt. Im HV trocknen. Ausbeute: 195mg ( 87% d.Th. ) dunkelrotes Pulver. ES⁻: M/e= 606.2 (M+-H), λₘₐₓ: 504.0.

### Beispiel 2

### (8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carbaldehyde (11)

1.) 50mg ( 82.3 µmol ) Tetrahydroxy Fredericamycin (Tetrol (10)) werden in 4ml DMF gelöst. Unter kräftigem Rühren wird einen wässrige Natriumjodat-Lösung ( 300mg NaIO4 in 1ml Wasser ) innerhalb einer Stunde zugetropft. Nach 1h rühren bei Raumtemperatur wird mit 2 Tropfen Trifluoressigsäure versetzt. Nach weiteren 30 Minuten Rühren wird die Reaktionslösung mit 3ml DMF verdünnt und anschließend mit 150mg NaIO₄ gelöst in 0.5ml Wasser versetzt.
   Nach einer weiteren Stunde trägt man in 100ml Wasser ein. Man saugt vom Niederschlag ab und trocknet im HV. Dunkelrotes Kristallpulver.
   Ausbeute: 41mg ( 100% d.Th. ). M/e= 501.3; UVₘₐₓ : 504,0nm
2.) 109mg ( 179 µmol ) Fredericamycin tetrol (9) werden in 8ml Pyridin gelöst. Zugabe von 180µl Wasser. In die Reaktionsmischung werden 450mg ( 1.08mmol, 6eq )
   ( Polystyrylmethyl )trimethylammonium perjodat Harz zugegeben. Anschließend lässt man 12h bei RT rühren. Man filtriert vom Harz ab, wäscht nach und engt zur Trockene ein. Dunkelroter Rückstand. Ausbeute 89.9mg( 100% d.Th. ).
   M/e=501.3; UVₘₐₓ: 504.0nm

### Beispiel 3

### (8S)-5-bromo-4',9.9'-trihydroxy-6'-methoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (2)

20mg ( 37.1µmol ) Fredericamycin (1) werden in 250µl DMF gelöst und anschließend bei 0°C innerhalb einer Stunde mit 6.3mg ( 35.3 µmol ) N-Bromsuccinimid in 250µl DMF versetzt. Die Reaktion rührt bei langsam auftauendem Eisbad über Nacht. Das DMF wird anschließend im Hochvakuum abgezogen und der Rückstand durch Präparative HPLC gereinigt.

Ausbeute: 7mg ( 32% d.Th. ) rote Kristallmasse. M/e = 616.1/618.1; λₘₐₓ = 486.0nm

### Beispiel 4

### (8S)-5-iodo-4',9.9'-trihydroxy-6'-methoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (3)

84mg ( 158µmol ) Fredericamycin (1) werden in 1.0 ml DMF gelöst und anschließend bei 0°C innerhalb einer Stunde mit 33.0 mg ( 150.0 µmol) N-Jodsuccinimid in 500µl DMF versetzt. Die Reaktion rührt bei langsam auftauendem Eisbad über Nacht. Das DMF wird anschließend im Hochvakuum abgezogen und der Rückstand ( 120mg (14) mit einem Gehalt von 80% durch präparative HPLC gereinigt ( Gradient CH₃CN 50 -90% innerhalb 16min ).

Ausbeute: 18mg ( 17% d.Th. ) rote Kristallmasse. M/e = 665.0; λₘₐₓ = 484.0nm

### Beispiel 5

### (8S)-4',9,9'-trihydroxy-5-bromo-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carbaldehyde (12)

100mg ( 200µmol) Fredericamycinaldehyd (11)werden unter Argon in 5ml DMF gelöst. Anschließend wird mit 200µl einer 1M Bromlösung in DMF versetzt. Nach 1.5h rühren bei RT werden noch einmal 20µl Bromlösung eingetragen. Die Reaktionsmischung ist laut HPLC nach insgesamt 3.5h vollständig.
In 150ml Wasser eintragen und mit Dichlormethan ausschütteln.

Ausbeute: 96mg ( 83% d.Th. ) dunkelrotes Pulver. M/e= 579/581, λₘₐₓ: 504.0.

### Beispiel 6

### (8S)-4',9,9'-trihydroxy-5-iodo-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carbaldehyde (13)

30mg ( 49 µmol ) Fredericamycin tetrol (10) werden in 1ml Dimethylsulfoxid/Wasser 9/1 gelöst. In die Reaktionsmischung werden 309mg ( 2.4mmol/g, 15eq ) ( Polystyrylmethyl )trimethylammonium perjodat Harz zugegeben. Anschließend lässt man 48h bei RT rühren. Man filtriert vom Harz ab, verdünnt mit Wasser und extrahiert 3x mit Dichlormethan dem 1% Trifluoreesigsäure zugestezt wurde. Nach den Trocknen engt man zur Trockene ein. Dunkelroter Rückstand ( HPLC-sauber ). Ausbeute 27.8mg( 90% d.Th. ). M/e=626.2; UVₘₐₓ: 500.0nm

### Beispiel 7

### (8S)-5-(trans-1-hexen-1yl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone (7)

10mg ( 15µmol ) Iodo Fredericamycin (3) werden unter Argon in 1ml DMF gelöst und anschließend mit 4.8mg (37.5µmol) trans 1-hexen-1yl-boronsäure (4), 0.9mg ( 0.78µmol ) Tetrakis(triphenyl) palladium (0) und 75µl (150µmol) 2M Na₂CO₃-Lösung versetzt. Man rührt 1h bei Raumtemperatur und erwärmt anschließend 12h auf 90°C. Die Reaktionsmischung wird zwischen Dichlormethan und 1N Salzsäure verteilt. Das Produkt wurde mittels präparativer HPLC ( RP18, CH₃CN-H₂O ) aufgereinigt.
Ausbeute: 4.5mg (48% d.Th.).

### Beispiel 8

### (8S)-5-phenyl-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone (8)

10mg ( 15µmol ) Iodo Fredericamycin (3) werden unter Argon in 1ml DMF gelöst und anschließend mit 4.6mg (37.7µmol) Phenylboronsäure (5), 0.9mg ( 0.78µmol ) Tetrakis ( triphenyl) palladium (0) und 75µl (150µmol) 2M Na₂CO₃-Lösung versetzt. Man rührt 1h bei Raumtemperatur und erwärmt anschließend 12h auf 90°C. Die Reaktionsmischung wird zwischen Dichlormethan und 1N Salzsäure verteilt. Der Rückstand wurde mittels präparativer HPLC aufgetrennnt.
(RP18, CH₃CN-H₂O )
Ausbeute: 4.0mg (43%d.Th.). M/e=615.0

### Beispiel 9

### (8S)-5-(4-fluorphenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone (9)

10mg ( 15µmol ) Iodo Fredericamycin (3) werden unter Argon in 1ml DMF gelöst und anschließend mit 5.3mg (37.8µmol) 4-Fluorphenylboronsäure (6), 1.0mg ( 0.87µmol ) Tetrakis(triphenyl) palladium (0) und 35.2mg (109µmol) Thalliumcarbonat versetzt. Man rührt 12h bei 90°C. Die Reaktionsmischung wird zwischen Dichlormethan und 1N Salzsäure verteilt und derer Rückstand wurde mittels präparativer HPLC aufgetrennt (RP18, CH₃CN-H₂O ). Ausbeute: 2.5mg (26%d.Th.). M/e=633.0

### Beispiel 10

In analoger Weise wie bei den obigen Beispiel können die folgenden Verbindungen hergestellt werden:

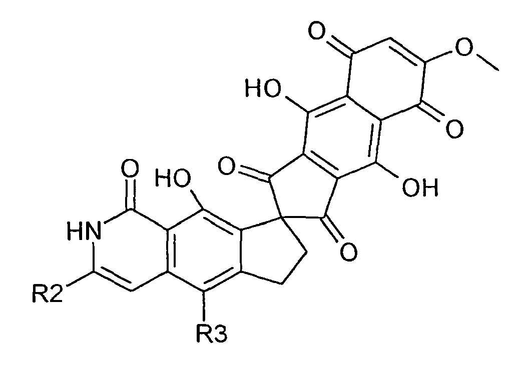

| Bsp. 10 | Name | R2 | R3 |
|---|---|---|---|
| A | (8S)-5-(3-pyridyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 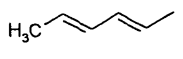 | 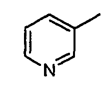 |
| B | (8S)-5-(4-pyridyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 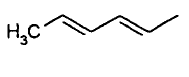 | 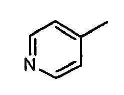 |
| C | (8S)-5-(5-indolyl)-4',9.9'-trihydroxy-6'-methoxy-3-[(1E,3E)-penta-1,3-dienyl]-6,7-dihydrospiro[cyclopenta[g]isoquin oline-8,2'-cyclopenta[b]-naphtalene] -1,1'-3',5',8'(2H)-pentone | 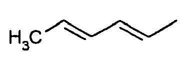 | 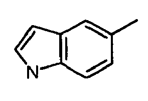 |
| D | (8S)-5-(4-dimethylaminophenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 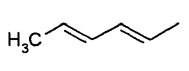 | 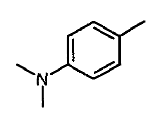 |
| E | (8S)-5-[4-(3,4-dimethylisoxazolyl)]-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 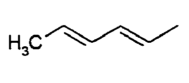 | 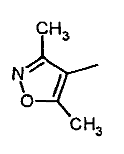 |
| F | (8S)-5-(3-furyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 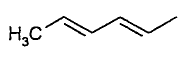 | 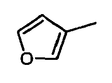 |
| G | (8S)-5-(4-benzyloxyphenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 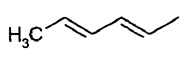 | 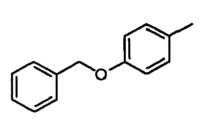 |
| H | (8S)-5-(4-methoxyphenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 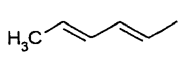 | 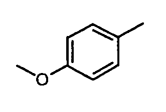 |
| I | (8S)-5-(2-thiophenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 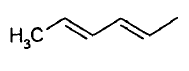 | 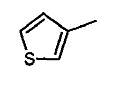 |
| J | (8S)-5-(3-thiophenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene]-1,1'-3',5',8'(2H)-pentone | 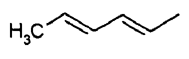 | 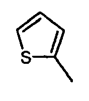 |
| K | (8S)-5-(4-carboxamidophenyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 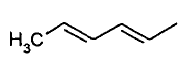 | 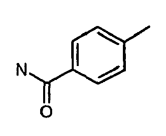 |
| L | (8S)-5-(1-dibenzofuranoyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 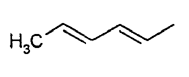 | 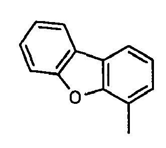 |
| M | (8S)-5-(2-N-methylpyrrolyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 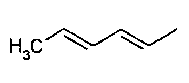 | 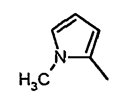 |
| N | (8S)-5-(2-pyridazinyl)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]-naphtalene] -1,1'-3',5',8'(2H)-pentone | 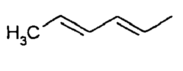 | 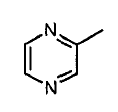 |
| O | (8S)-5-(phenyl)-4',9,9',-tri-hydroxy-6'-methoxy-1,1',3',5',8'-pentaoxol,1',2,3',5',6,7,8'-octahydrospiro[cyclopenta[g]isoquinoline-8,2'-cyclopenta[b]naphthalene]-3-carbaldehyde O-methyloxime | 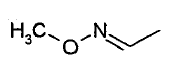 | 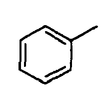 |
| P | (8S)-5-(2-thiophenyl)-4',9,9',-trihydroxy-6'-methoxy-1,1',3',5',8'-pentaoxo-1,1',2,3',5',6,7,8'-octahydrospiro[cyclopenta[g]isoquinoline-8,2'-cyclopenta[b]-naphthalene]-3-carbaldehyde O-methyloxime | 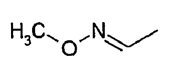 | 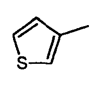 |

### Beispiel 11

### Wasserlöslichkeit der Fredericamycin-Derivate

Die Wasserlöslichkeit der verschiedenen Fredericamycin-Derivate kann in 0.9 %iger NaCl-Lösung mit einem pH-Wert von 7 bestimmt werden.

## Patentansprüche

1. Verbindungen gemäß allgemeiner Formel Ia oder Ib: 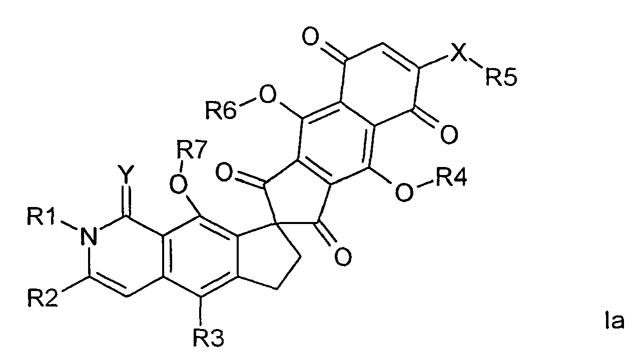 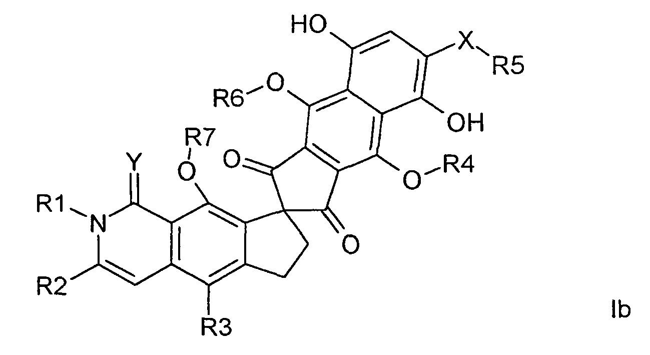 wobei jeweils
R1 H, C₁-C₆-Alkyl, Cycloalkyl, C₁-C₄-Alkyl-Cycloalkyl,
R2 C₁-C₁₄-Alkyl, C₂-C₁₄-Alkenyl, 1,3-Butadienyl, 1-Butan, C₁-C₄-Alkyl-Aryl,Heteroaryl, C₁-C₄-Alkyl-Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0,1,2, CH₂SCOR21, CH₂OSO₂-R21, CHO, CH=NOH, CH(OH)R21, -CH=NOR21, -CH=NOCOR21, -CH=NOCH₂CONR21R22, -CH=NOCH(CH₃)CONR21R22, -CH=NOC(CH₃)₂CONR21R22, -CH=N-NHCO-R23, -CH=N-NHCO-CH₂NHCOR21, -CH=N-O-CH₂NHCOR21, -CH=N-NHCS-R23, -CH=CR24R25 ( trans oder cis ), COOH, COOR21, CONR21R22, -CH=NR21, -CH=N-NR21R22, 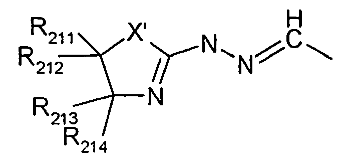 (mit X' = NR215, O, S und R211, R212, R213, R214, R215 unabhängig voneinander H oder C₁-C₆-Alkyl), -CH=N-NHSO₂-Aryl,
-CH=N-NHSO₂-Heteroaryl,
R21, R22 unabhängig voneinander C₁-C₁₄-Alkyl, C₁-C₁₄-Alkanoyl, C₁-C₆-Alkylhydroxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbidungen (wie z.B. Glucose, Desoxyglucose, Ribose, Desoxyribose),
R23 unabhängig von R21, die selben Bedeutungen wie R21 oder CH₂pyridinium-salze, CH2tri-C₁-C₆-alkylammonmium-salze,
R24 unabhängig von R21, die selben Bedeutungen wie R21 oder H, CN, COCH₃, COOH, COOR21, CONR21R22, NH₂, NHCOR21
R25 unabhängig von R21, die selben Bedeutungen wie R21 oder H, CN, COCH₃, COOH, COOR21, CONR21R22, NH₂, NHCOR21
R24,R25 zusammen C₄-C8-Cycloalkyl,
R3 C₂-C₁₄-Alkyl, C₂-C₁₄-Alkenyl, C₂-C₁₄-Alkinyl, Aryl, C₁-C₄-Alkyl-Aryl, Heteroaryl, C₁-C₄-Alkyl-Heteroaryl, wobei die Aryle oder Herteroaryle mit einem weiteren Aryl, C₁-C₄-Alkyl-Aryl, O-Aryl, C₁-C₄-Alkyl-O-Aryl, Heteroaryl, C₁-C₄-Alkyl-Heteroaryl, O-Heteroaryl oder C₁-C₄-Alkyl-O-Heteroaryl substituiert sein können, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 2 bis 6, für o = 1,-1, p = 1 bis 2m+o; für m = 4 bis 6, o = -3, p = 1 bis 2m+o; Y = unabhängig von einander ausgewählt aus der Gruppe Halogen, OH, OR31, NH₂, NHR31, NR31R32, SH, SR31), CH₂NHCOR31, CH₂NHCSR31, CH₂S(O)nR31 mit n=0,1,2, CH₂SCOR31, CH₂OSO₂-R31, CHO, CH=NOH, CH(OH)R31, -CH=NOR31, -CH=NOCOR31, -CH=NOCH₂CONR31R32, -CH=NOCH(CH₃)CONR31R32, -CH=NOC(CH₃)₂CONR31R32, -CH=N-NHCO-R33, -CH=N-NHCO-CH₂NHCOR31, -CH=N-O-CH₂NHCOR31, -CH=N-NHCS-R33, -CH=CR34R35 ( trans oder cis ), COOH, COOR31, CONR31R32, -CH=NR31, -CH=N-NR31R32, 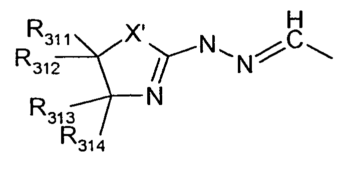 (mit X' NR315, O, S und R311, R312, R313, R314, R315 unabhängig voneinander H oder C₁-C₆-Alkyl), -CH=N-NHSO₂-Aryl, -CH=N-NHSO₂-Heteroaryl,
R31, R32 unabhängig voneinander C₁-C₁₄-Alkyl, C₁-C₁₄-Alkanoyl, C₁-C₆-Alkylhydroxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbidungen (wie z.B. Glucose, Desoxyglucose, Ribose, Desoxyribose),
R33 unabhängig von R31, die selben Bedeutungen wie R31 oder CH₂pyridinium-salze, CH2tri-C₁-C₆-alkylammonmium-salze,
R34 unabhängig von R21, die selben Bedeutungen wie R31 oder H, CN, COCH₃, COOH, COOR21, CONR31R32, NH₂, NHCOR31
R35 unabhängig von R31, die selben Bedeutungen wie R31 oder H, CN, COCH₃, COOH, COOR31, CONR31R32, NH₂, NHCOR31
R34, R35 zusammen C₄-C₈-Cycloalkyl,
R5 H, C₁-C₆-Alkyl, Cycloalkyl, C₁-C₄-Alkyl-Cycloalkyl, Heterocycloalkyl, C₁-C₄-Alkyl-Heterocycloalkyl, Aryl, C₁-C₄-Alkyl-Aryl, Heteroaryl, C₁-C₄-Alkyl-Heteroaryl,
R4,R6,R7 unabhängig voneinander H, C₁-C₆-Alkyl, CO-R41
R41 unabhängig von R21, die selben Bedeutungen wie R21
X O, S, NH, N-R8, wobei R8 unabhängig von R5 die gleichen Bedeutung wie R5 annehmen kann oder R5 und R8 zusammen mit dem N eine 4, 5, 6, 7 oder 8 gliedrigen Heteroycloalkylring bilden, der optional noch ein weiteres Heteroatom ausgewählt aus der Gruppe N, O, S enthalten kann,
oder X-R5 zusammen gleich H,
Y O, S, NR9, wobei R9 Hoder C₁-C₆-Alkyl sein kann,
bedeutet, deren Stereoisomere, Tautomere und deren physiologisch verträglichen Salze oder Einschlussverbindungen.

2. Verbindungen gemäß Anspruch 1, wobei Formel I a oder I b, die Stereochemie von Formel II a oder II b 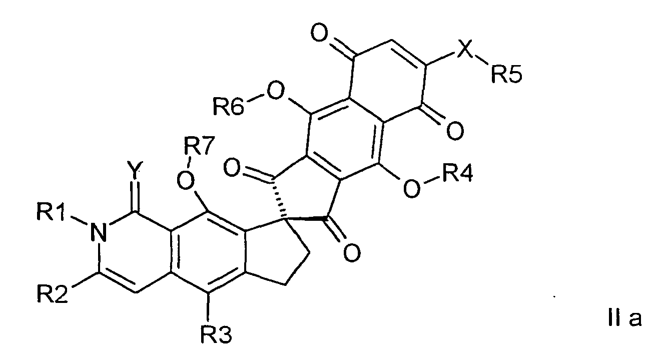 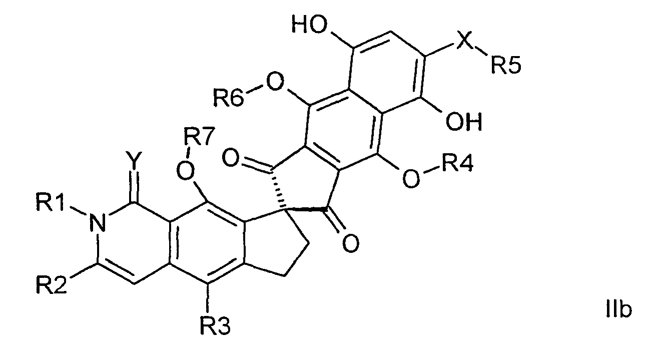 annimmt.

3. Verbindungen der allgemeinen Formel Ia, Ib, IIa oder IIb gemäß Anspruch 1 oder 2, bei denen die Reste R außer R3, die in den vorhergehenden Ansprüchen angegebenen Bedeutungen haben und R3 gegenüber R3 gleich H die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestensverfünfzigfacht, insbesondere verhundertfacht, oder sogar verfünfhundertfacht mittels Einführung von Gruppen, die vermehrt Wasserstoffbrückenbindungen ausbilden können, und/oder polar und / oder ionisch sind.

4. Verbindungen der allgemeinen Formel Ia, Ib, IIa oder IIb gemäß Anspruch 1 oder 2, bei denen die Reste R außer R2, die in den vorhergehenden Ansprüchen angegebenen Bedeutungen haben und R2 gegenüber R2 gleich CH=CH-CH=CH-CH3 die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht, mittels Einführung von Gruppen, die vermehrt Wasserstoffbrückenbindungen ausbilden können, und/oder polar und / oder ionisch sind.

5. Verbindungen gemäß einem der Ansprüche 1 bis 4, wobei
R1 H, C₁-C₅-Alkyl, Cycloalkyl, insbesondere H,
R2 C₁-C₅-Alkyl, C₁-C₄-Alkyl-Aryl, C₂-C₅-Alkenyl, Heteroaryl, C₁-C₄-Alkyl-Heteroaryl, CHF₂, CF₃, Polyolseitenkette insbesondere CHOH-CHOH-CHOH-CHOH-CH₃, CHOH-CHOH-CH=CH-CH₃, CH=CH-CHOH-CHOH-CH₃, CH₂Y (Y=F,Cl,Br,I), ), CH₂NH₂, CH₂NR21R22, CH₂NHCOR23, CH₂NHCSR23, CH₂SH, CH₂S(O)nR21 mit n=0,1,2, CH₂SCOR21, insbesondere CH₂OH, CH₂OR21, CH₂OSO₂-R21, insbesondere CHO, CH(OR21)₂, CH(SR21)₂, CN, CH=NOH, CH=NOR21, CH=NOCOR21, CH=N-NHCO-R23, CH=CR24,R25 ( trans oder cis ), insbesondere COOH (insbesondere deren physiologisch verträglichen Salze), COOR21, CONR21R22, -CH=NR21, -CH=N-NR21R22, 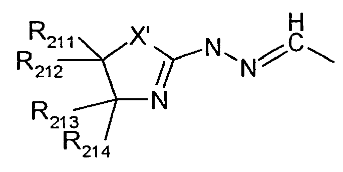 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl, CH=N-NHCO-R23,
R21, R22 unabhängig voneinander C₁-C₆-Alkyl, Cycloalkyl, Aryl, C₁-C₄-Alkyl-Aryl, Heteroaryl, C₁-C₄-Alkyl-Heteroaryl
R23 unabhängig von R21, die selben Bedeutungen wie R21 oder CH₂pyridinium-salze, CH2tri-C₁-C₆-alkylammonmium-salze,
R24 unabhängig von R21, die selben Bedeutungen wie R21 oder H, CN, COCH₃, COOH, COOR21, CONR21R22, NH₂, NHCOR21
R25 unabhängig von R21, die selben Bedeutungen wie R21 oder H, CN, COCH₃, COOH, COOR21, CONR21R22, NH₂, NHCOR21
R24,R25 zusammen C₄-C8-Cycloalkyl,
R3 C₂-C₁₄-Alkyl, C₂-C₁₄-Alkenyl, C₂-C₁₄-Alkinyl, Aryl, C₁-C₄-Alkyl-Aryl, Heteroaryl, C₁-C₄-Alkyl-Heteroaryl, wobei die Aryle oder Herteroaryle mit einem weiteren Aryl, C₁-C₄-Alkyl-Aryl, O-Aryl, C₁-C₄-Alkyl-O-Aryl, Heteroaryl, C₁-C₄-Alkyl-Heteroaryl, O-Heteroaryl oder C₁-C₄-Alkyl-O-Heteroaryl substituiert sein können,
R5 H, C₁-C₃-Alkyl, Cycloalkyl
R4,R6,R7 unabhängig voneinander H, C₁-C₅-Alkyl, CO-R41
R41 unabhängig von R21, die selben Bedeutungen wie R21
X O, S, NH, N-R8
Y O, S, NH
bedeutet.

6. Verbindungen gemäß einem der Ansprüche 1 bis 5 in der Form von Einschlussverbindungen mit Cyclodextrin, insbesondere alpha-Cyclodextrin.

7. Arzneimittel enthaltend Verbindungen nach einem der Ansprüche 1 bis 6 neben den üblichen Träger und Hilfsstoffen.

8. Arzneimittel nach Anspruch 7 in Kombination mit weitere Wirkstoffen zur Tumorbehandlung.

9. Verwendung von Verbindungen gemäß einem der Ansprüche 1 bis 6 zur Herstellung von Arzneimitteln zur Behandlung von Tumoren, insbesondere von solchen, die durch die Inhibierung der Topoisomerasen I und/oder II behandelt werden können, verwendet.

10. Verwendung von Verbindungen gemäß einem der Ansprüche 1 bis 6 zur Herstellung von Arzneimitteln zur Behandlung von Parasiten.

11. Verwendung von Verbindungen gemäß einem der Ansprüche 1 bis 6 zur Herstellung von Arzneimitteln zur Immunsupression.

12. Verwendung von Verbindungen gemäß einem der Ansprüche 1 bis 6 zur Herstellung von Arzneimitteln zur Behandlung von Neurodermitis.

## Claims

1. The compounds according to the general formula Ia or Ib: 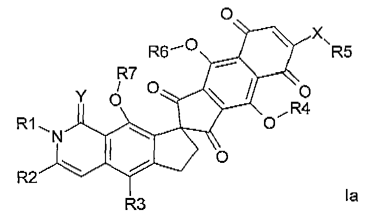 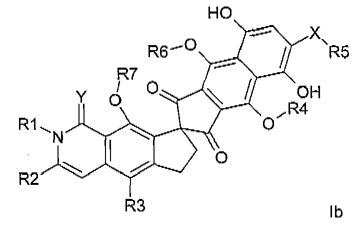 wherein in each,
R1 means H, C₁-C₆ alkyl, cycloalkyl, C₁-C₄ alkylcycloalkyl,
R2 means C₁-C₁₄ alkyl, C₂-C₁₄ alkenyl, 1,3-butadienyl, 1-butane, C₁-C₄ alkylaryl, heteroaryl, C₁-C₄ alkyl heteroaryl, cycloalkyl, C₁-C₄ alkyl-cycloalkyl, heterocycloalkyl, C₁-C₄ alkylheterocycloalkyl, CₘH₂ₘ₊ₒ₋ₚYₚ (with m = 1 to 6, for o = 1, p = 1 to 2m+o; for m = 2, to 6, o = -1, p = 1 to 2m+o; for m = 4 to 6, o = -2, p = 1 to 2m+o; Y = independently from each other selected from the group consisting of halogen, OH, OR21, NH₂, NHR21, HR21R22, SH, SR21), CH₂NHCOR21, CH₂NHCSR21, CH₂S(O)nR21, with n = 0, 1, 2, CH₂SCOR21, CH₂OSO₂-R21, CHO, CH=NOH, CH(OH)R21, -CH=NOR21, -CH=NOCOR21, -CH=NOCH₂CONR21R22, - CH=NOCH(CH₃)CONR21R22, -CH=NOC(CH₃)₂CONR21R22, -CH=N-NHCO-R23, - CH=N-NHCO-CH₂NHCOR21, -CH=N-O-CH₂NHCOR21, -CH=N-NHCS-R23, - CH=CR24R25 (trans or cis), COOH, COOR21, CONR21R22, -CH=NR21, -CH=N-NR21R22, 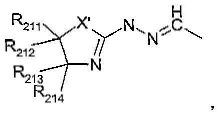 (with X' = NR215, O, S, and R211, R212, R213, R214, R215 being independently from each other H or C₁-C₆ alkyl), -CH=N-NHSO₂ aryl, -CH=N-NHSO₂ heteroaryl,
R21, R22 are independently from each other C₁-C₁₄ alkyl, C₁-C₁₄ alkanoyl, C₁-C₆ alkylhydroxy, C₁-C₆ alkylamino, C₁-C₆ alkylamino-C₁-C₆ alkyl, C₁-C₆ alkylamino-di-C₁-C₆ alkyl, cycloalkyl, C₁-C₄ alkylcycloalkyl, heterocycloalkyl, C₁-C₄ alkylheterocycloalkyl, aryl, aryloyl, C₁-C₄ alkylaryl, heteroaryl, heteroaryloyl, C₁-C₄ alkylheteroaryl, cycloalkanoyl, C₁-C₄ alkanoylcycloalkyl, heterocycloalkanoyl, C₁-C₄ alkanoylheterocycloalkyl, C₁-C₄ alkanoylaryl, C₁-C₄ alkanoylheteroaryl, mono- and di-sugar residues linked through a C atom which would carry an OH residue in the sugar, wherein the sugars are independently from each other selected from the group consisting of glucuronic acid and its stereo isomers at all optical atoms, aldopentoses, aldohexoses, including their dcsoxy compounds (such as e.g. glucose, desoxyglucose, ribose, desoxyribose),
R23 independently of R21, has the same meanings as R21, or CH₂-pyridinium salts, CH₂-tri-C₁-C₆ alkylammonium salts,
R24 independently of R21, has the same meanings as R21, or H, CN, COCH₃, COOH, COOR21, CONR21R22, NH₂, NHCOR21,
R25 independently of R21, has the same meanings as R21, or H, CN, COCH₃, COOH, COOR21, CONR21R22, NH₂, NHCOR21,
R24, R25 together mean C₄-C₈ cycloalkyl,
R3 means C₂-C₁₄ alkyl, C₂-C₁₄ alkenyl, C₂-C₁₄ alkinyl, aryl, C₁-C₄ alkylaryl, heteroaryl, C₁-C₄ alkylheteroaryl, wherein the aryls or heteroaryls may be substituted with another aryl, C₁-C₄ alkylaryl, O-aryl, C₁-C₄ alkyl-O-aryl, heteroaryl, C₁-C₄ alkylheteroaryl, O-heteroaryl or C₁-C₄ alkyl-O-heteroaryl, cycloalkyl, C₁-C₄ alkylcycloalkyl, heterocycloalkyl, C₁-C₄ alkylheterocycloalkyl, CₘH₂ₘ₊ₒ₋ₚYₚ (with m = 2 to 6, for o = 1, -1, p = 1 to 2m+o; for m = 4 to 6, o = -3, p = 1 to 2m+o; Y = independently from each other selected from the group consisting of halogen, OH, OR31, NH₂, NHR31, NR31R32, SH, SR31), CH₂NHCOR31, CH₂NHCSR31, CH₂S(O)nR31, with n= 0, 1, 2, CH₂SCOR31, CH₂OSO₂-R31, CHO, CH=NOH, CH(OH)R31, -CH=NOR31, -CH=NOCOR31, -CH=NOCH₂CONR31R32, - CH=NOCH(CH₃)CONR31R32, -CH=NOC(CH₃)₂CONR31 R32, -CH=N-NHCO-R33, - CH=N-NHCO-CH₂NHCOR31, -CH=N-O-CH₂NHCOR31, -CH=N-NHCS-R33, - CH=CR34R35 (trans or cis), COOH, COOR31, CONR31R32, -CH=NR31, -CH=N-NR31R32, 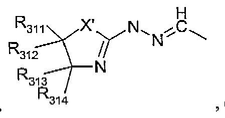 (with X' = NR315, O, S, and R311, R312, R313, R314, R315 being independently from each other H or C₁-C₆ alkyl), -CH=N-NHSO₂ aryl, -CH=N-NHSO₂- heteroaryl,
R31, R32 mean independently from each other C₁-C₁₄ alkyl, C₁-C₁₄ alkanoyl, C₁-C₆ alkylhydroxy, C₁-C₆ alkylamino, C₁-C₆ alkylamino-C₁-C₆ alkyl, C₁-C₆ alkylamino-di-C₁-C₆ alkyl, cycloalkyl, C₁-C₄ alkylcycloalkyl, heterocycloalkyl, C₁-C₄ alkylheterocycloalkyl, aryl, aryloyl, C₁-C₄ alkylaryl, heteroaryl, heteroaryloyl, C₁-C₄ alkylheteroaryl, cycloalkanoyl, C₁-C₄ alkanoylcycloalkyl, heterocycloalkanoyl, C₁-C₄ alkanoylheterocycloalkyl, C₁-C₄ alkanoylaryl, C₁-C₄ alkanoylheteroaryl, mono- and di-sugar residues linked through a C atom which would carry an OH residue in the sugar, wherein the sugars are independently from each other selected from the group consisting of glucuronic acid and its stereo isomers at all optical atoms, aldopentoses, aldohexoses, including their desoxy compounds (such as e.g. glucose, desoxyglucose, ribose, desoxyribose),
R33 independently of R31, has the same meanings as R31, or CH₂-pyridinium salts, CH₂-tri-C₁-C₆ alkylammonium salts,
R34 independently of R21, has the same meanings as R31, or H, CN, COCH₃, COOH, COOR21, CONR31R32, NH₂, NHCOR31,
R35 independently of R31, has the same meanings as R31, or H, CN, COCH₃, COOH, COOR31, CONR31R32, NH₂, NHCOR31,
R34, R35 together mean C₄-C₈ cycloalkyl,
R5 means H, C₁-C₆ alkyl, cycloalkyl, C₁-C₄ alkylcycloalkyl, heterocycloalkyl, C₁-C₄ alkylheterocycloalkyl, aryl, C₁-C₄ alkylaryl, heteroaryl, C₁-C₄ alkylheteroaryl,
R4, R6, R7 independently from each other mean H, C₁-C₆ alkyl, CO-R41,
R41 independently from R21, has the same meanings as R21,
X means O, S, NH, N-R8, wherein R8 independently from R5 may adopt the same meaning as R5, or R5 and R8, together with the N, form a ring with 4, 5, 6, 7, or 8 members, which may optionally contain still another Heteroatom selected from the group N, O, S,
or X-R5 may together be H,
Y means 0, S, NR9, wherein R9 may be H or C₁-C₆ alkyl,
as well their stereoisomers, tautomers, and their physiologically tolerable salts or inclusion compounds.

2. The compounds according to claim 1, wherein Formula Ia or Ib adopt the stereochemistry of Formula IIa or IIb

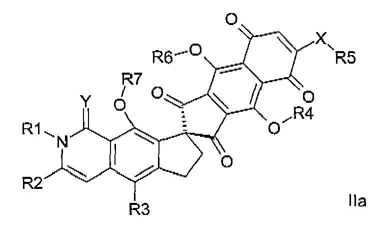

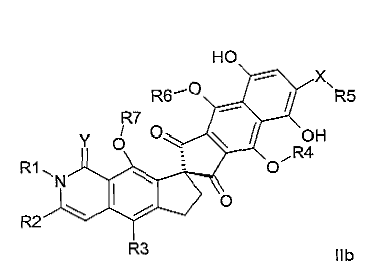

3. The compounds of the general Formula Ia, Ib, IIa, IIb according to claim 1 or 2, wherein the residues R, except R3, have the meanings indicated in the previous claims, and wherein R3 has a water solubility that is at least two times higher, preferably at least five times higher, more preferred at least ten times higher, particularly preferred at least fifty times higher, particularly hundred times higher, or even five hundred times higher compared to R3 being H, with all other residues being maintained by means of groups, which can form more hydrogen bond, and/or being polar and/or ionic.

4. The compounds of the general Formula Ia, Ib, IIa, IIb according to claim 1 or 2, wherein the residues R, except R2, have the meanings indicated in the previous claims, and wherein R2 has a water solubility that is at least two times higher, preferably at least five times higher, more preferred at least ten times higher, particularly preferred at least fifty times higher, particularly hundred times higher, or even five hundred times higher compared to R2 being CH=CH-CH=CH-CH₃, with all other residues being maintained by means of groups, which can form more hydrogen bond, and/or being polar and/or ionic.

5. The compounds according to one of the claims 1 to 5, wherein
R1 means H, C₁-C₅ alkyl, cycloalkyl, especially H,
R2 means C₁-C₅ alkyl, C₁-C₄ alkylaryl, C₂-C₅ alkenyl, heteroaryl, C₁-C₄ alkylheteroaryl, CHF₂, CF₃, polyol side chain, particularly CHOH-CHOH-CHOH-CHOH-CH₃, CHOH-CHOH-CH=CH-CH₃, CH=CH-CHOH-CHOH-CH₃, CH₂Y (Y = F, Cl, Br, I), CH₂NH₂, CH₂NR21R22, CH₂NHCOR23, CH₂NHCSR23, CH₂SH, CH₂S(O)nR21, with n = 0, 1, 2, CH₂SCOR21, particularly CH₂OH, CH₂OR21, CH₂OSO₂-R21, particularly CHO, CH(OR21)₂, CH(SR21)₂, CN, CH=NOH, CH=NOR21, CH=NOCOR21, CH=N-NHCO-R23, CH=CR24, R25 (trans or cis), particularly COOH (particularly their physiologically tolerable salts), COOR21, CONR21R22, -CH=NR21, -CH=N-NR21R22, 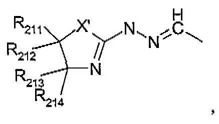 (with X' =NR215, O, S, and R211, R212, R213, R214, R215 being independently from each other H or C₁-C₆ alkyl), -CH=N-NHSO₂ aryl, -CH=N-NHSO₂ heteroaryl, CH=N-NHCO-R23,
R21, R22 independently from each other mean C₁-C₆ alkyl, cycloalkyl, aryl, C₁-C₄ alkylaryl, heteroaryl, C₁-C₄ alkylheteroaryl,
R23 independently of R21, has the same meanings as R21, or CH₂-pyridinium salts, CH₂-tri-C₁-C₆ alkylammonium salts,
R24 independently of R21, has the same meanings as R21, or H, CN, COCH₃, COOH, COOR21, CONR21R22, NH₂, NHCOR21,
R25 independently of R21, has the same meanings as R21, or H, CN, COCH₃, COOH, COOR21, CONR21R22, NH₂, NHCOR21,
R24, R25 together mean C₄-C₈ cycloalkyl,
R3 means C₂-C₁₄ alkyl, C₂-C₁₄ alkenyl, C₂-C₁₄ alkinyl, aryl, C₁-C₄ alkylaryl, heteroaryl, C₁-C₄ alkylheteroaryl, wherein the aryls or heteroaryls may be substituted with another aryl, C₁-C₄ alkylaryl, O-aryl, C₁-C₄ alkyl-O-aryl, heteroaryl, C₁-C₄ alkylheteroaryl, O-heteroaryl or C₁-C₄ alkyl-O-heteroaryl,
R5 means H, C₁-C₃ alkyl, cycloalkyl,
R4, R6, R7 independently from each other mean H, C₁-C₅ alkyl, CO-R41,
R41 independently from R21, has the same meanings as R21,
X means O, S, NH, N-R8,
Y means O, S, NH.

6. The compounds according to one of the claims 1 to 5 in the form of their inclusion compounds with cyclodextrin, particularly alpha cyclodextrin.

7. Drugs containing compounds according to one of the claims 1 to 6, as well as the usual carrier and adjuvants.

8. Drugs according to claim 7 in combination with further agents for tumor treatment.

9. The use of compounds according to one of the claims 1 to 6 for preparation of drugs for tumor treatment, particularly of those that can be treated by inhibition of the topoisomerases I and/or II.

10. The use of compounds according to one of the claims 1 to 6 for preparation of drugs for treatment of parasites,

11. The use of compounds according to one of the claims 1 to 6 for preparation of drugs for immunosuppression.

12. The use of compounds according to one of the claims 1 to 6 for preparation of drugs for treatment of atopic dermatitis.

## Revendications

1. Composés selon la formule générale Ia ou Ib : 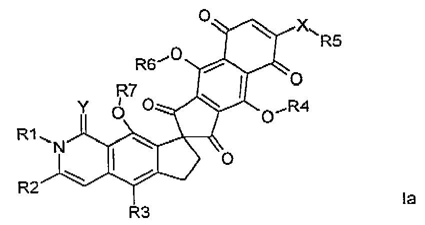 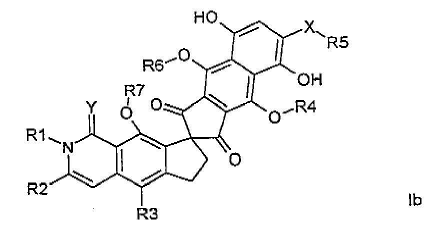 dans lesquelles, respectivement
R1 représente H, alkyle en C₁-C₆, cycloalkyle, alkyle en C₁-C₄-cycloalkyle,
R2 représente alkyle en C₁-C₁₄, alcényle en C₂-C₁₄, 1,3-butadiènyle, 1-butane, alkyle en C₁-C₄-aryle, hétéroaryle, alkyle en C₁-C₄-hétéroaryle, cycloalkyle, alkyle en C₁-C₄-cycloalkyle, hétérocycloalkyle, alkyle en C₁-C₄-hétérocycloalkyle, CₘH₂ₘ₊ₒ₋ₚYₚ (avec m = 1 à 6, pour o = 1, p = 1 à 2m+o ; pour m = 2 à 6, o = -1, p = 1 à 2m+o ; pour m = 4 à 6, o = -2, p = 1 à 2m+o ; Y = choisi indépendamment l'un de l'autre dans le groupe constitué par halogène, OH, OR21, NH₂, NHR21, NR21R22, SH, SR21), CH₂NHCOR21, CH₂NHCSR21, CH₂S(O)nR21 avec n = 0, 1, 2, CH₂SCOR21, CH₂OSO₂-R21, CHO, CH=NOH, CH(OH)R21, -CH=NOR21, -CH=NOCOR21, -CH=NOCH₂CONR21R22, -CH=NOCH(CH₃)CONR21R22, -CH=NOC(CH₃)₂CONR21R22, -CH=N-NHCO-R23, -CH=N-NHCO-CH₂NHCOR21, -CH=N-O-CH₂NHCOR21, - CH=N-NHCS-R23, -CH=CR24R25 (trans ou cis), COOH, COOR21, CONR21R22, -CH=NR21, -CH=N-NR21R22, 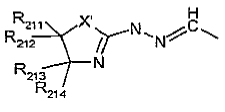 (avec X' = NR215, O, S et R211, R212, R213, R214, R215 qui représentent indépendamment les uns des autres H ou alkyle en C₁-C₆), -CH=N-NHSO₂-aryle, -CH=N-NHSO₂-hétéroaryle,
R21, R22 représentent indépendamment l'un de l'autre alkyle en C₁-C₁₄, alcanoyle en C₁-C₁₄, alkylhydroxy en C₁-C₆, alkylamino en C₁-C₆, alkylamino en C₁-C₆-alkyle en C₁-C₆, alkylamino en C₁-C₆-di-alkyle en C₁-C₆, cycloalkyle, alkyle en C₁-C₄-cycloalkyle, hétérocycloalkyle, alkyle en C₁-C₄-hétérocycloalkyle, aryle, aryloyle, alkyle en C₁-C₄-aryle, hétéroaryle, hétéroaryloyle, alkyle en C₁-C₄-hétéroaryle, cycloalcanoyle, alcanoyle en C₁-C₄-cycloalkyle, hétérocycloalcanoyle, alcanoyle en C₁-C₄-hétérocycloalkyle, alcanoyle en C₁-C₄-aryle, alcanoyle en C₁-C₄-hétéroaryle, résidus de mono- et disaccharides, qui sont reliés par le biais d'un atome de C qui porterait un groupe OH dans le sucre, où les sucres sont choisis indépendamment les uns des autres dans le groupe constitué de l'acide glucuronique et ses stéréoisomères dans tous les atomes de C optiques, les aldopentoses, les aldohexoses, y compris leurs composés désoxy (comme par exemple le glucose, le désoxyglucose, le ribose, le désoxyribose),
R23 a, indépendamment de R21, les mêmes significations que R21 ou représente des sels de CH₂-pyridinium, des sels de CH₂-tri-C₁-C₆-alkylammonium,
R24 a, indépendamment de R21, les mêmes significations que R21 ou représente H, CN, COCH₃, COOH, COOR21, CONR21R22, NH₂, NHCOR21,
R25 a, indépendamment de R21, les mêmes significations que R21 ou représente H, CN, COCH₃, COOH, COOR21, CONR21R22, NH₂, NHCOR21,
R24, R25 représentent conjointement cycloalkyle en C₄-C₈,
R3 représente alkyle en C₁-C₁₄, alcényle en C₂-C₁₄, alcinyle en C₂-C₁₄, aryle, alkyle en C₁-C₄-aryle, hétéroaryle, alkyle en C₁-C₄-hétéroaryle ; où les aryles et hétéroaryles peuvent être substitués par un autre aryle, alkyle en C₁-C₄-aryle, O-hétéroaryle ou alkyle en C₁-C₄-O-hétéroaryle; cycloalkyle, alkyle en C₁-C₄-cycloalkyle, hétérocycloalkyle, alkyle en C₁-C₄-hélérocycloalkyle, CₘH₂ₘ₊ₒ₋ₚYₚ (avec m = 2 à 6, pour o = 1, -1, p = 1 à 2m+o; pour m = 4 à 6, o = -3, p = 1 à 2m+o; Y = choisi indépendamment l'un de l'autre dans le groupe constitué par halogène, OH, OR31, NH₂, NHR31, NR31R32, SH, SR31), CH₂NHCOR31, CH₂NHCSR31, CH₂S(O)nR31 avec n = 0, 1, 2, CH₂SCOR31, CH₂OSO₂-R31, CHO, CH=NOH, CH(OH)R31, -CH=NOR31, -CH=NOCOR31, -CH=NOCH₂CONR31R32, -CH=NOCH(CH₃)CONR31R32, -CH=NOC(CH₃)₂CONR31R32, -CH=N-NHCO-R33, -CH=N-NHCO-CH₂NHCOR31, -CH=N-O-CH₂NHCOR31, - CH=N-NHCS-R33, -CH=CR34R35 (trans ou cis), COOH, COOR31, CONR31R32, -CH=NR31, -CH=N-NR31R32, 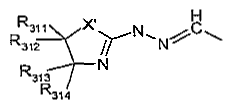 (avec X' = NR315, 0, S et R311, R312, R313, R314, R315 qui représentent indépendamment les uns des autres H ou alkyle en C₁-C₆), -CH=N-NHSO₂-aryle, -CH=N-NHSO₂-hétéroaryle,
R31, R32 représentent indépendamment l'un de l'autre alkyle en C₁-C₁₄, alcanoyle en C₁-C₁₄, alkylhydroxy en C₁-C₆, alkylamino en C₁-C₆, alkylamino en C₁-C₆-alkyle en C₁-C₆, alkylamino en C₁-C₆-di-alkyle en C₁-C₆, cycloalkyle, alkyle en C₁-C₄-cycloalkyle, hétérocycloalkyle, alkyle en C₁-C₄-hétérocyloalkyle, aryle, aryloyle, alkyle en C₁-C₄-aryle, hétéroaryle, hétéroaryloyle, alkyle en C₁-C₄-hétéroaryle, cycloalcanoyle, alcanoyle en C₁-C₄-cycloalkyle, hétérocycloalcanoyle, alcanoyle en C₁-C₄-hétérocycloalkyle, alcanoyle en C₁-C₄-aryle, alcanoyle en C₁-C₄-hétéroaryle, résidus de mono- et disaccharides, qui sont reliés par le biais d'un atome de C qui porterait un groupe OH dans le sucre, où les sucres sont choisis indépendamment les uns des autres dans le groupe constitué de l'acide glucuronique et ses stéréoisomères dans tous les atomes de C optiques, les aldopentoses, les aldohexoses, y compris leurs composés désoxy (comme par exemple le glucose, le désoxyglucose, le ribose, le désoxyribose),
R33 a, indépendamment de R31, les mêmes significations que R31 ou représente des sels de CH₂-pyridinium, des sels de CH₂-tri-C₁-C₆-alkylammonium,
R34 a, indépendamment de R21, les mêmes significations que R31 ou représente H, CN, COCH₃, COOH, COOR21, CONR31R32, NH₂, NHCOR31,
R35 a, indépendamment de R31, les mêmes significations que R31 ou représente H, CN, COCH₃, COOH, COOR31, CONR31R32, NH₂, NHCOR31,
R34, R35 représentent conjointement cycloalkyle en C₄-C₈,
R5 représente H, alkyle en C₁-C₆, cycloalkyle, alkyle en C₁-C₄-cycloalkyle, hétérocycloalkyle, alkyle en C₁-C₄-hétérocyloalkyle, aryle, alkyle en C₁-C₄-aryle, hétéroaryle, alkyle en C₁-C₄-hétéroaryle,
R4, R6, R7 représentent indépendamment les uns des autres H, alkyle en C₁-C₆, CO-R41
R41 a, indépendamment de R21, les mêmes significations que R21
X représente O, S, NH, N-R8, où R8, indépendamment de R5, peut prendre les mêmes significations que R5 ou R5 et R8 forment conjointement avec N un cycle hétérocycloalkyle à 4, 5, 6, 7 ou 8 membres, qui peut éventuellement encore contenir un hétéroatome choisi dans le groupe N, O, S,
ou X-R5 représentent conjointement H,
Y représente O, S, NR9, où R9 peut être H ou alkyle en C₁-C₆, leurs stéréoisomères, tautomères et leurs sels physiologiquement acceptables ou composés d'inclusion.

2. Composés selon la revendication 1, dans lesquels la formule Ia ou Ib prend la stéréochimic de la formule IIa ou IIb

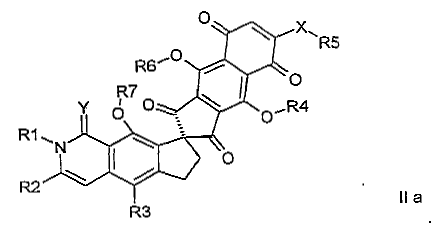

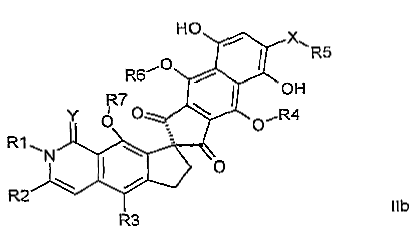

3. Composés de formule générale Ia, Ib, IIa ou IIb selon la revendication 1 ou 2, dans lesquels les radicaux R en dehors de R3 ont les significations indiquées dans les revendications précédentes et R3 par rapport à R3 égal H au moins multiplie par deux, de préférence au moins multiplie par cinq, plus préférentiellement au moins multiplie par dix, particulièrement préférentiellement au moins multiplie par cinquante, en particulier multiplie par cent voire même multiplie par cinq cents la solubilité tout en conservant tous les autres radicaux, au moyen de l'introduction de groupes qui peuvent former des liaisons de ponts hydrogène de manière démultipliée et/ou sont polaires et/ou ioniques.

4. Composés de formule générale Ia, Ib, IIa ou IIb selon la revendication 1 ou 2, dans lesquels les radicaux R en dehors de R2 ont les significations indiquées dans les revendications précédentes et R2 par rapport à R2 égal CH=CH-CH=CH-CH₃ au moins multiplie par deux, de préférence au moins multiplie par cinq, plus préférentiellement au moins multiplie par dix, particulièrement préférentiellement au moins multiplie par cinquante, en particulier multiplie par cent voire même multiplie par cinq cents la solubilité tout en conservant tous les autres radicaux, au moyen de l'introduction de groupes qui peuvent former des liaisons de ponts hydrogène de manière démultipliée et/ou sont polaires et/ou ioniques.

5. Composés selon l'une des revendications 1 à 4, dans lesquels
R1 représente H, alkyle en C₁-C₅, cycloalkyle, en particulier H,
R2 représente alkyle en C₁-C₅, alkyle en C₁-C₄-aryle, alcényle en C₂-C₅, hétéroaryle, alkyle en C₁-C₄-hétéroaryle, CHF₂, CF₃, chaîne latérale de polyole notamment CHOH-CHOH-CHOH-CHOH-CH₃, CHOH-CHOH-CH=CH-CH₃, CH=CH-CHOH-CHOH-CH₃, CH₂Y (Y=F, Cl, Br, I), CH₂NH₂, CH₂NR21NR22, CH₂NHCOR23, CH₂NHCSR23 CH₂SH, CH₂S(O)nR21 avec n=0, 1, 2, CH₂SCOR21, en particulier CH₂OH, CH₂OR21, CH₂OSO₂R21, en particulier CHO, CH(OR21)₂, CH(SR21)₂, CN, CH=NOH, CH=NOR21, CH=NOCOR21, CH=N-NHCO-R23, CH=CR24, R25 (trans ou cis), en particulier COOH (notamment ses sels physiologiquement acceptables), COOR21, CONR21R22, -CH=NR21, -CH=N-NR21R22, 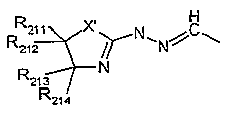 (avec X' = NR215, O, S et R211, R212, R213, R214, R215 qui représentent indépendamment les uns des autres H ou alkyle en C₁-C₆), -CH=N-NHSO₂-aryle, -CH=N-NHSO₂-hétéroaryle, CH=N-NHCO-R23,
R21, R22 représentent indépendamment l'un de l'autre alkyle en C₁-C₆, cycloalkyle, aryle, alkyle en C₁-C₄-aryle, hétéroaryle, alkyle en C₁-C₄-hétéroaryle,
R23 a, indépendamment de R21, les mêmes significations que R21 ou représente des sels de CH₂-pyridinium, des sels de CH₂-tri-C₁-C₆-alkylammonium,
R24 a, indépendamment de R21, les mêmes significations que R21 ou représente H, CN, COCH₃, COOH, COOR21, CONR21R22, NH₂, NHCOR21,
R25 a, indépendamment de R21, les mêmes significations que R21 ou représente H, CN, COCH₃, COOH, COOR21, CONR21R22, NH₂, NHCOR21,
R24, R25 représentent conjointement cycloalkyle en C₄-C₈,
R3 représente alkyle en C₂-C₁₄, alcényle en C₂-C₁₄, alcinyle en C₂-C₁₄, aryle, alkyle en C₁-C₄-aryle, hétéroaryle, alkyle en C₁-C₄-hétéroaryle ; où les aryles et hétéroaryles peuvent être substitués par un autre aryle, alkyle en C₁-C₄-aryle, O-aryle, alkyle en C₁-C₄-O-aryle, hétéroaryle, alkyle en C₁-C₄-hétéroaryle, O-hétéroaryle ou alkyle en C₁-C₄-O-hétéroaryle,
R5 représente H, alkyle en C₁-C₃, cycloalkyle,
R4, R6, R7 représentent indépendamment les uns des autres H, alkyle en C₁-C₅, CO-R41
R41 a, indépendamment de R21, les mêmes significations que R21
X représente O, S, NH, N-R8,
Y représente O, S, NH.

6. Composés selon l'une des revendications 1 à 5 sous la forme de composés d'inclusion avec de la cyclodextrine, notamment l'alpha-cyclodextrine.

7. Médicament contenant des composés selon l'une des revendications 1 à 6 en plus des véhicules et auxiliaires usuels.

8. Médicament selon la revendication 7 en combinaison avec d'autres principes actifs pour le traitement des tumeurs.

9. Utilisation des composés selon l'une des revendications 1 à 6 pour la préparation de médicaments pour le traitement des tumeurs, notamment de celles qui peuvent être traitées par l'intermédiaire de l'inhibition des topoisomérases I et/ou II.

10. Utilisation des composés selon l'une des revendications 1 à 6 pour la préparation de médicaments pour le traitement des parasites.

11. Utilisation des composés selon l'une des revendications 1 à 6 pour la préparation de médicaments pour l'immunosuppression.

12. Utilisation des composés selon l'une des revendications 1 à 6 pour la préparation de médicaments pour le traitement de la neurodermite.
